# EUROPEAN PATENT SPECIFICATION

(11) **EP 1 334 197 B1**
(45) Date of publication and mention of the grant of the patent: **05.01.2011**
(21) Application number: 01980767.6
(22) Date of filing: 12.11.2001
(51) Int. Cl.: C12N 15/40, A61K 39/12, C12N 15/81, C07K 14/08, A61K 39/39, A61P 31/12, C12N 1/19, A61K 39/295

(54) **YEAST DERIVED VACCINE AGAINST IPNV**
VAKZINE GEGEN IPNV DIE AUS HEFEZELLEN ISOLIERT WERDEN
VACCIN DERIVE DE LA LEVURE CONTRE IPNV

(30) Priority: 11.11.2000 GB 0027644; 14.12.2000 GB 0030765
(43) Date of publication of application: 13.08.2003
(73) Proprietor: The University Court of The University of Aberdeen, Aberdeen AB24 3FX (GB)
(72) Inventor: MELVIN, William, Thomas, Aberdeen, Grampian AB15 7PQ (GB); BREEMAN, Suzanne, Ellon, Aberdeenshire AB41 8AL (GB); LABUS, Marie, Beagley, Inverurie, Grampian AB51 5QN (GB)
(74) Representative: Kremer, Simon Mark
(86) International application number: PCT/GB2001/004986
(87) International publication number: WO 2002/038770

(56) References cited:
- US-A- 5 165 925
- FROST PETTER ET AL: "Analysis of the antibody response in Atlantic salmon against recombinant VP2 of infectious pancreatic necrosis virus (IPNV)." FISH & SHELLFISH IMMUNOLOGY, vol. 8, no. 6, August 1998 (1998-08), pages 447-456, XP002194211 ISSN: 1050-4648
- CHRISTIE K E: "Immunization with viral antigens: Infectious pancreatic necrosis." DEVELOPMENTS IN BIOLOGICAL STANDARDIZATION, vol. 90, 1997, pages 191-199, XP001062658 Meeting;Oslo, Norway; June 5-7, 1996, 1997 S. Karger AG; S. Karger AG P.O. Box, Allschwilerstrasse 10, CH-4009 Basel, Switzerland; New York, New York, USA ISBN: 3-8055-6482-1 cited in the application
- HAVARSTEIN L S ET AL: "SEQUENCE OF THE LARGE DOUBLE-STRANDED RNA SEGMENT OF THE N1 STRAIN OF INFECTIOUS PANCREATIC NECROSIS VIRUS A COMPARISON WITH OTHER BIRNAVIRIDAE" JOURNAL OF GENERAL VIROLOGY, vol. 71, no. 2, 1990, pages 299-308, XP001062695 ISSN: 0022-1317
- SAELENS XAVIER ET AL: "Protection of mice against a lethal influenza virus challenge after immunization with yeast-derived secreted influenza virus hemagglutinin." EUROPEAN JOURNAL OF BIOCHEMISTRY, vol. 260, no. 1, February 1999 (1999-02), pages 166-175, XP002194212 ISSN: 0014-2956
- LABUS MARIE B ET AL: "Antigenic comparison of a truncated form of VP2 of infectious pancreatic necrosis (IPN) virus expressed in four different cell types." FISH & SHELLFISH IMMUNOLOGY, vol. 11, no. 3, April 2001 (2001-04), pages 203-216, XP002194213 ISSN: 1050-4648 cited in the application

## Description

The present invention relates to vaccine compositions to protect fish against infectious pancreatic necrosis virus.

### Background art

Infectious pancreatic necrosis virus (IPNV) is an unenveloped, icosahedral, bisegmented dsRNA virus, and causes a highly contagious disease of young hatchery-reared salmon [1,2] as well as other farmed fish [3]. This disease, once established, is very difficult to eradicate from infected fish, and the development of a safe, efficient, and inexpensive vaccine against IPNV is very much needed. The outbreak of such disease brings about very serious economic damage to fish farmers.

IPNV has one major structural protein, VP2 (52kD), and three other proteins, VP1 (90kD), VP3 (30kD) and VP4 (28kD) [4-7]. VP1 is a putative viral polymerase [8], while VP2 is the major outer capsid protein [9]. VP3 is a further capsid protein and VP4 has been regarded as a cleaved form of VP3 during viral maturation [9,10].

The nucleotide and amino acid sequences for VP2 and VP3 are well known in the art see e.g. Havarstein et al (1990) "Sequence of the large double-stranded RNA segment of the N1 strain of the infectious pancreatic necrosis virus: a comparison with other Birnaviridae" J Gen Virol 71: 299-308.

It is also known that there are strain variations, see e.g. Pryde et al, 1992 Archives of Virology 129, 287-293.

Currently available vaccines against IPNV include inactivated IPN virus, which is grown in fish cell lines and then inactivated using a standard viral inactivant. However, the large scale production of vaccines from fish cell lines can be costly. There is also a risk of reversion of the virus to the virulent form.

WO94/04565 of Proteus House relates to a synthetic peptide having at least one antigenic property of a strain of IPNV, wherein the peptide consists substantially of a selected amino acid sequence. Peptides were synthesised using solid phase chemistry.

WO 99/50419 (University of Maryland) relates to methods for preparing a non-pathogenic infectious pancreatic necrosis virus, comprising various steps which lead to NS-protein deficient IPNV. These virions were intended for use as live attenuated vaccines.

In addition recombinant protein vaccines are discussed in various documents.

Christie (1997),Fish Vaccinology, Dev Biol Stand. Basel, Karger, vol 90, pp 191-199, eds Gudding et al) discusses a vaccine produced in *E.coli* which contains recombinant VP2 protein.

US 5165925 (University of Oregon) discusses methods for immunising fish against the VR-299 and SP serotypes of IPNV, wherein the vaccine consists essentially of a polypeptide from the viral A segment and including at least VP2, having been expressed in a bacterial host.

In addition Korean patent KR100227102 also relates to antigens from IPNV and related cDNA and vaccines

Labus et al (2001) Fish & Shellfish Immunology 11: 203-216, which was published after the presently claimed priority dates, compares the antigenicity of structural proteins from IPNV when prepared in different host systems including bacteria and yeast. One of these proteins was VP2-trunc, said to encompass residues of 147-307 of VP2. The most authentic folding was believed to occur in CHSE (Chinook Salmon Embryo) and CHO (Chinese Hamster Ovary) cells.

Frost et al "Analysis of the antibody response in Atlantic salmon against recombinant VP2 of infectious pancreatic necrosis virus (IPNV)" Fish & Shellfish Immunology, Volume 8, Issue 6, August 1998, Pages 447-456 describes an IPNV-specific humoral immune response in Atlantic salmon pre-smolts and in rabbits immunised with purified E. coli-expressed recombinant VP2.

Notwithstanding the above disclosures there is an ongoing need for novel vaccine preparations which are effective against IPNV.

### Disclosure of the invention

The present inventors have cloned and expressed IPNV antigens in the yeast strain *Pichia pastoris*. These expressed recombinant proteins were then used as a vaccine preparation in Atlantic salmon (Salmo salar). As shown in the Examples, such vaccine preparations appear to be highly effective. Surprisingly, as shown in the Examples hereinafter, it is believed they may be considerably more effective than available preparations from bacteria.

In other aspects of the invention as defined in the claims specific combinations of effective antigens, particularly bivalent vaccines, (for example VP3 and a VP2 protein, generally referred to herein as VP2var) are also provided, as are fusions of these and methods of producing and using them.

Preferred vaccines are as defined in the claims and consist essentially of VP3 and VP2var polypeptides, and are capable of inducing immunity in fish to the subsequent infection by the IPNV, said polypeptides having been produced in a yeast host by an expression vector compatible with the host, the expression vectors including an inserted DNA sequence from IPNV viral DNA coding for the IPNV polypeptide in the vaccine.

Various aspects and embodiments of the present invention are set out in the claims herein. The invention will now be discussed in more detail.

In one aspect of the present invention there is disclosed a process for producing a bivalent vaccine composition as defined in claim 1 e.g. for use in Atlantic salmon (*Salmo* salar) which process comprises expressing a polypeptide encoding an IPNV protein (e.g. from the IPNV strain Sp) in a yeast strain (e.g. *Pichia pastoris*, preferably *P. pastoris* GS115) and formulating the polypeptide as a vaccine.

Described herein are processes which comprise the steps of:
(i) isolating one or more IPNV coding regions,
(ii) preparing a recombinant plasmid containing the IPNV coding regions (i.e. one suitable for expression in yeast cell lines, which plasmid encodes one or more IPNV polypeptide or polypeptides)
(iii) preparing yeast cell lines expressing the IPNV polypeptides (e.g. by electroporation, optionally separate plasmids in separate host cells),
(iv) screening for expression of the IPNV polypeptides in the cell lines,
(v) immunising one or more fish with cell lines expressing the IPNV polypeptides (or preparations of the polypeptides therefrom).

The preferred IPNV proteins, combinations of proteins, and fusions are discussed in more detail below. Most preferred are VP3 and VP2var (a smaller region of the whole VP2 protein which shows the highest degree of amino acid variation between strains).

Preferably the method comprises expressing two different IPNV proteins in *Pichia pastoris* such as to produce a bivalent vaccine

Generally speaking, in the light of the disclosure of the present invention, those skilled in the art are will be able to construct appropriate vectors and design protocols for recombinant gene expression. Suitable vectors can be chosen or constructed, containing appropriate regulatory sequences, including promoter sequences (see below), terminator fragments, polyadenylation sequences, enhancer sequences, marker genes, signal sequences and other sequences as appropriate. For further evidence of the common general knowledge see, for example, Molecular Cloning: a Laboratory Manual: 2nd edition, Sambrook et al, 1989, Cold Spring Harbor Laboratory Press (or later editions of this work). Many known techniques and protocols for manipulation of nucleic acid, for example in preparation of nucleic acid constructs, mutagenesis (see above discussion in respect of variants), sequencing, introduction of DNA into cells and gene expression, and analysis of proteins, are described in detail in Current Protocols in Molecular Biology, Second Edition, Ausubel et al. eds., John Wiley & Sons, 1992. The disclosures of Sambrook et al. and Ausubel et al. are incorporated herein by reference.

The expressed polypeptide is secreted from the host cell so the vector preferably includes a signal sequence to direct the protein so that it is secreted from the cell. A preferred signal sequence is the yeast α mating factor signal sequence.

In one aspect of the present invention, there is provided an appropriate IPNV-expressing yeast vector as defined in claim 15.

"Vector" is defined to include, inter alia, any plasmid, cosmid or phage in double or single stranded linear or circular form which may or may not be self transmissible or mobilizable, and which can transform a prokaryotic or eukaryotic host either by integration into the cellular genome or exist extrachromosomally (e.g. autonomous replicating plasmid with an origin of replication).

The vector may be a bi-functional expression vector which functions in multiple hosts. In the case of cDNA this may be under the control of an appropriate promoter or other regulatory elements for expression in the host cell.

A preferred vector is the *Pichia pastoris* expression vector, pPICZαB.

In a further aspect of the invention, there is disclosed a yeast host cell containing or transformed with a heterologous vector according to the present invention. As is well known to those skilled in the art, the term "heterologous" is used broadly in this aspect to indicate that the IPNV sequence of nucleotides in question have been introduced into the yeast cell, or a progenitor cell thereof, using genetic engineering, i.e. by human intervention. Nucleic acid heterologous to the host cell will be non-naturally occurring in the host cell type.

The polypeptide may be partially purified from the host before being used as a vaccine. Where the polypeptide is secreted from the host cell, the cells may be separated from the media by centrifugation, the cells being pelleted and the media being the supernatant. In such a situation, the supernatant, which contains the secreted polypeptide, may be used directly as a vaccine, or in a vaccine composition. Alternatively, the polypeptide may be partially purified from this supernatant, for example using affinity chromatography.

The method may further comprise admixing the partially purified polypeptide with another component, such as another polypeptide and/or an adjuvant, diluent or excipient as described below.

Vaccine preparations as defined in claim 17 form one aspect of the present invention.

In one aspect of the present invention the vaccine produced in the yeast such as *Pichia* is based on VP2 and VP3 or immunogenic combinations or fragments of both.

In the case of fragments, in each case, the vaccine polypeptides disclosed herein may comprise, consist of, or consist essentially of the fragment in question.

In a preferred VP2-based embodiments, the fragment is VP2var. This is a smaller region of the VP2 protein previously identified as a variable segment of VP2 comprising approximately 150 amino acids (amino acids 183-337; 678-1140 nt ) (Havarstein, et al, 1990 Journal of General Virology 71, 299-308). The protein is discussed in the Pryde et al (1992) study supra which compared the sequence from a 192 amino acid stretch of VP2 isolated from a Scottish IPNV strain (Sp serotype)against the same region of a field isolate from Shetland (Sh serotype), a Norwegian strain (N1 serotype) and a Canadian strain (Ja serotype). The Sh strain differed from Sp by 1 amino acid substitution, the N1 strain by 2 substitutions and the Ja by 33 substitutions.

Preferred primers for use in the methods of the present invention are specific for the polypeptides in question, and preferably are those which introduce restriction sites for cloning and\or avoid GC rich sequences. For example:

559                                586
CTA ACA ACG GAA TTC ATG GAC AAA GTC   VP2var forward primer (SEQ ID.
NO. 1)
            EcoRI site

A preferred primer for use with a secretion signal vector is as follows:
5' gaagctgcagaggacaaagtcaac3' VP2var forward (SEQ ID. NO. 2)

A further preferred primer is:

CGT TGC CGA TTG GCG GCC GCT GGT TGA TC  VP2var reverse primer (SEQ
ID. NO. 3)
NotI site

The sequences obtainable by using SEQ ID. NO. 1 and 2 respectively with SEQ ID. NO. 3 are given as Sequence Annex A.

In another embodiment preferred primers are:
5' accactgcagtcacagtcctgaatc3' VP2var forward (SEQ ID. NO. 4)
5' gagcgcggccgccgcaattccgttccctg3' VP2var reverse (SEQ ID. NO. 5)

The sequence obtainable by using SEQ ID. NO. 4 and 5 is given as Sequence Annex B.

Thus there is disclosed a vaccine composition comprising a polypeptide consisting of the amino acid sequence of VP2var obtainable using any of these primers, corresponding to any of these regions, or having any of the sequences given in Annex A or B.

Embodiments of this aspect may comprises a VP2 polypeptide as described above in combination with a VP3 polypeptide or fragment. For instance as obtainable by use of the following primers:

2305 2330
CCT GGG ACT GCA GAT GGC ATC AAA TG VP3 forward primer (SEQ
ID. NO. 6)
PstI site

GTT ACA CCG CGG CCG CGT CTC CGC TGG G        VP3 reverse primer (SEQ
ID. NO. 7)
            NotI site

The sequence obtainable by using SEQ ID. NO. 6 and 7 is given as Sequence Annex C.

Other preferred primers for use with a secretion signal vector are as follows:
5' gacgctgcagtgcaacgcctcctg 3' VP3 forward (SEQ ID. NO. 8)
5' gtgcagcggccgccgggggtcgtcgtttcatc 3' VP3reverse 2936-2967 (SEQ ID. NO. 9)

The sequence obtainable by using SEQ ID. NO. 8 and 9 is given as Sequence Annex D.

Thus there is provided a vaccine composition comprising a polypeptide consisting of the amino acid sequence of VP3 obtainable using any of these primers, corresponding to any of these regions, or having any of the sequences given in Annex C or D.

Sequences may be obtained using primers from base sequences according to methods well known to those skilled in the art. A typical method may use 1ng of purified template DNA containing the coding regions for VP2 and VP3, 25pmoles each PCR primer in 45µl of PCR master mix containing 2.5mM MgCl₂ (Advanced Biotechnologies). Cycling may be carried out e.g. in a Perkin Elmer Thermocycler using the following cycling parameters; 94°C for 5 min followed by 35 cycles of 30s at 48°C, 1min 20s at 72°C, 30s at 94°C and a final incubation of 10min at 72°C. A 10µl aliquot of the resultant PCR reactions is then electrophoretically separated through a 1.5% agarose gel containing 0.5µg/ml ethidium bromide.

In another embodiment of the present invention provides for the production of IPNV vaccines as defined in the claims by combination of one or more copies of each of (all or part of) the VP2 or VP3 antigens, fused together in the correct orientation for expression as a single polypeptide. For example, this may comprise a fusion protein derived from at least one copy of each of the VP2 and VP3 protein sequences joined end to end. To achieve this, multiple copies of the coding region from the relevant IPNV genes are ligated together to form a single open reading frame with a single initiation and termination codon. This is cloned into a suitable expression vector and recombinantly produced as previously described. The resulting "multivalent IPNV antigen" contains at least one copy of each of the antigenic protein sequences contained within both the IPNV antigens, and is therefore a more potent stimulator of a host immune response.

In yet another embodiment of the invention, the IPNV vaccine as defined in the claims is comprised of a novel combination of two or more copies of either the VP2 or VP3 antigens, fused together in the correct orientation for expression as a single polypeptide. For example, this may comprise a fusion protein derived from 2 copies of the VP3 protein sequence joined end to end, or two copies of the VP2 protein joined end to end. To achieve this, multiple copies of the coding region from the relevant IPNV gene are ligated together to form a single open reading frame with a single initiation and termination codon. This is cloned into a suitable expression vector and recombinantly produced as previously described. This resulting "multimeric IPNV antigen" contains multiple copies of the antigenic protein sequence-contained within the IPNV antigen, and is therefore a more potent stimulator of a host immune response.

Preferred primers for use with a secretion signal vector are as follows:
5' gacgctgcagtgcaacgcctcctg 3' VP3 forward (SEQ ID. NO. 10)
5' ctctctagagtctccgctggg 3' VP3 reverse (SEQ ID. NO. 11)
5' ccctcagagtcacagtcctg 3' VP2var forward (SEQ ID. NO. 12)
5' gagcgcggccgccgcaattccgttccctg3' VP2var reverse (SEQ ID. NO. 13)

The amplified nucleotide sequences, when ligated together, code for a hybrid protein consisting of amino acids of VP3 fused to amino acids of VP2. This is shown as Annex E.

Vaccines produced in yeast may therefore include (comprise, consist of, or consist essentially of) any one or more of the IPNV polypeptides discussed above and described in Sequence Annexes hereto. The polypeptides need not be in pure form, provided that they are capable of conferring a protective response in a fish into which they are introduced.

The vaccine is a bivalent vaccine comprising polypeptides derived from the IPNV virus, preferably as shown in any of Sequence Annexes A-E.

Thus vaccines comprise two polypeptides derived from the IPNV virus based on VP2var and VP3. These two polypeptides may be expressed as a fusion. Indeed it is believed that such bivalent vaccines are particularly effective and such preferred combination vaccines (howsoever produced, although preferably produced in a yeast such as *Pichia*) form one particular aspect of the invention.

Minor variants of the above sequences may be employed in particular embodiments as described hereinafter.

Vaccines may contain other bacterial antigens used to control other diseases i.e. vaccine composition may be included within a multivalent vaccine which includes antigens against other diseases of fish.

Thus a preferred multivalent injection vaccine may contain the two IPN proteins discussed above, plus antigens to other fish diseases such as *Aeromonas salmonicida* (Strain MT004) and\or *Aeromonas salmonicida* (Strain MT423) [see EP 0587636 of the Secretary of State for Scotland]. Also one or more *Vibrio* antigens (including *V.anguillarum, V. salmonicida* and *V. viscosus*) antigens e.g. inactivated *Vibrio anguillarum* (Strain 78-SKID); Inactivated *Vibrio anguillarum* (Strain MSC 275); Inactivated *Vibrio salmonicida* (Strain VS 855); Inactivated *Vibrio viscosus* (Strain HW/98/7/2)

IPNV proteins when used in the present invention may be fused to other sequences.

For example, The polypeptide may be in the form of a fusion protein, for example it may be linked to a leader or signal sequence as discussed above. Such a sequence may, for example cause the expressed protein to be secreted from the host cell. Such a sequence may be cleaved off from the rest of the polypeptide before the polypeptide is formulated into a composition, or may be retained on the polypeptide in the composition. Preferably, the signal sequence is the yeast α-mating factor signal sequence. Preferably the signal sequence is not cleaved from the expressed polypeptide, but is retained on the polypeptide and thereby forms part of the vaccine.

Polypeptides disclosed herein may be linked to a suitable carrier e.g. to enhance immunogenicity. Suitable carriers include bovine serum albumin, keyhole limpet haemocyanin etc.

The polypeptide may be attached to a linker polypeptide, which linker peptide links the polypeptide to a particles such as latex or bentonite. This may facilitate its administration as an immersion vaccine (see below). Linker polypeptides may comprise functional domains such as acidic blob domains from eukaryotic transcription factors, or histone protein polybasic domains.

In addition to the polypeptides, the vaccine composition may further comprise a pharmologically acceptable diluent, buffer, adjuvant, or excipient, or combination of these. Such materials should be nontoxic and should not interfere with the efficacy of the active ingredient. The precise nature of the carrier or other material will depend on the route of administration, which may be oral, or by injection, e.g. intravenous.

Microparticle formulations which may be used in the present invention include biodegradable microspheres composed of polymer materials such as polyester poly(lactide-co-glycolide) (PLG) (Eldridge et al, Molec Immunol 28: 287-294 (1991), and atelocollagen (Fujioka et al, J. Control. Release 33: 307-315 (1995). Injection of atelocollagen "Minipellets" containing a plasmid encoding human HST I/FGF-4 has been shown to result in slow release of DNA and subsequent prolonged expression of functional protein in mice (Ochiya et al, Nature Medicine 5: 707-710 (1999)). PLG microspheres are sufficiently robust to survive ingestion and arrive intact at the gut-associated lymphoid tissue (Eldridge et al, Adv Exp Med Biol 251: 191-202 (1989)), and have been used to introduce recombinant antigens and attenuated viruses into mammals by both systemic and oral routes (O'Hagan et al, Immunology 73: 239-242 (1991); O'Hagan et al, Vaccine 11: 149-154 (1993); Marx et al, Science 260: 1323-1328 (1993); Jones et al, Vaccine 15: 814-817 (1997)). In fish orally incubated with human gamma globulin incorporated into PLG microparticles, uptake of the foreign protein into intestinal tissues and the kidneys was demonstrable (O'Donnell et al, Fish & Shellfish Immunol. 6: 507-520 (1996)).

Thus the present invention also relates to methods for formulation of such proteins to render them suitable for administration by immersion or orally via incorporation into fish food. In one embodiment of the invention pertaining to formulation of the vaccine for the immersion vaccination of fish, the recombinant proteins are packaged within a micro-particulate delivery system, which may include, but is not limited to, latex beads, poly(lactide-co-glycolide) microspheres, atelocollagen "minipellets", bentonite, orporous apatite ceramics including hypoxyapatite (HA) and beta-tricalcium phosphate (TCP).

Suitable materials are well known to the person skilled in the art. Examples include; water, saline (e.g. 0.85% sodium chloride; see Ph.Eur. monograph 2001:0062), buffered saline, fish oil with an emulsifier (e.g. a lecithin, Bolec MT), inactivant (e.g. formaldehyde; see Ph.Eur. monograph 1997:0193), mineral oils, such as light mineral oils, alhydrogel, aluminium hydroxide. Where used herein, the term "oil adjuvant" to embraces both mineral oils and synthetic oils. A preferred adjuvant is Montanide ISA 711 (SeppicQuai D'OrsaY, 75321 Paris, France) which is a manide oleate in an oil suspension.

For example, for an immersion vaccine, an aqueous suspension is preferred. For an oral vaccine a fish oil and lecithin carrier system is preferred. For an injection vaccine Montanide ISA711®, Sepic at a ratio of 30:70 is preferred.

Preferred doses range from 50 to 150 µg antigen per fish, more preferably 70 to 125 µg per fish. A preferred dose is about 100µg per fish.

For injection most preferred is dosage unit comprising 105µg of each of the VP2 and VP3 antigen in 100 µl i.e. 1.05 g/l of each protein.

For oral use emulsion is added to fish feed pellets and and fed over a 10 day feeding period to deliver an equivalent of 100 µl of vaccine.

Those skilled in the art are well aware of typical modes of administration. For example:

A vaccine composition may be administered orally or by injection.

As is well known in the art, one preferred mode of administration is comprises the use of oral vaccination technologies whereby vaccine is administered in fish food, or by direct addition of vaccine to the water in which the fish swim ("immersion vaccination"). Optionally this may be used in revaccination in order to boost immunity established by other means (Dunn et al, Aquacultural Engineering 9: 23-32 (1990); Ellis, Fish Pathology 30:293-300 (1995). Thus in certain embodiments, microparticles such as those discussed above are administered by immersion of the aquaculture species in a suspension fluid containing the microparticles at an appropriate concentration, or by incorporation into fish food.

A vaccine composition may be administered alone or in combination with other treatments, either simultaneously or sequentially.

A vaccine composition may be administered as a course of a number of discrete doses over a period of time. For example it may be administered over a period of around fourteen days.

Vaccination may be repeated at daily, twice-weekly, weekly or monthly intervals. For example a boost vaccination may be administered after the initial dose. For example a boost may be administered at around fourteen weeks after the vaccination. The initial vaccination and any boost may be carried out using the same or different modes of administration. For example, the initial may be by injection and the boost may be by oral administration. A preferred regime includes a first vaccination by injection, followed by (14 weeks post challenge) a two week course of orally administered boost vaccine, or a booster prior to an expected IPN outbreak (e.g. just after transfer to seawater).

In practice, microparticles containing recombinant protein are diluted to a suitable concentration in an enclosed tank containing water as used for the normal culturing of the relevant fish species and fish fry are immersed in this solution for a period of several hours. The fish are then returned to their normal culturing conditions. With this practice the recombinant proteins may enter the gills or digestive tract of the fish and be engulfed by antigen presenting cells and subsequently induce an immune response.

Alternatively, microparticles containing the recombinant proteins are incorporated into a typical fish food preparation and fed to fish in place of ordinary feed. In this method the recombinant proteins will enter-the digestive tract stimulating an immune response in systemic or gut-associated lymphoid tissues. This method has the advantage of being suitable for use in netted enclosures where sealed tanks are not available.

Other adjuvants, carriers etc., and modes of administration may be found by referring to Gudding et al (1999) Veterinary immunology and Immunopathology 72, 203-212.

The polypeptides (including variants, derivatives, fusions and conjugates) described herein may also be used in methods of diagnosis for IPNV, and such use of the polypeptides and diagnostic methods constitute further aspects of the invention. For example, the polypeptides may be used as a substrate to screen for antibodies in a fish and thereby determine whether or not the fish has been infected with IPNV. Such an assay could be by ELISA, or other technique as would be understood by the person skilled in the art.

The polypeptides including variants, derivatives, fusions and conjugates) described herein may also be used in the manufacture of a vaccine or other medicament for treatment of, or having prophylactic effect against IPNV.

Also disclosed herein is a method of therapeutic treatment or prophylaxis of IPNV, comprising administering a vaccine composition as described herein to a fish.

Also disclosed herein is a fish population which has been treated or immunized with a vaccine or composition described elsewhere herein.

The IPNV preparations described herein e.g. produced recombinantly in yeast by expression from encoding nucleic acid therefor, may be used to raise antibodies employing techniques which are standard in the art. Such antibodies may function *in vivo* as protective (neutralising) antibodies, or may be isolated e.g. for use in ELISA

As discussed above, embodiments of the present invention also embrace processes, methods and vaccine (compositions) based upon polypeptides which are variants (fragment, derivative or homologue etc.) of the sequences of VP2var or VP3 given herein. The variant may be capable of stimulating the production of antibodies which bind IPNV, these antibodies may be neutralizing antibodies.

The production of antibodies which bind IPNV, or which neutralize IPNV may be assessed by ELISA or by neutralization assays, respectively. Appropriate assays are described elsewhere herein.

Artificial variants (derivatives) may be prepared by those skilled in the art, for instance by site directed or random mutagenesis of a nucleic acid encoding the polypeptide shown in a sequence Annex, the variant polypeptide may then be produced by expression from a suitable host, e.g., *Pichia pastoris* as described elsewhere herein. Alternatively the variant maybe produced by direct synthesis.

Preferably the variant polypeptide is generated either directly or indirectly (e.g. via one or amplification or replication steps) from an original nucleic acid encoding all or part of the sequences shown in a sequence Annex.

Homology (i.e. similarity or identity) may be as defined using sequence comparisons are made using FASTA and FASTP (see Pearson & Lipman, 1988. Methods in Enzymology 183: 63-98). Parameters are preferably set, using the default matrix, as follows: Gapopen (penalty for the first residue in a gap): -12 for proteins; Gapext (penalty for additional residues in a gap): -2 for proteins; KTUP word length: 2 for proteins. The amino acid sequence shares at least 90%, 95%, 96%, 97%, 98% or 99% identity with the sequences shown herein.

In addition to one or more changes within the amino acid sequence shown, a variant polypeptide may include additional amino acids at the C-terminus and/or N-terminus.

Changes may be desirable for a number of reasons, including introducing or removing the following features: sites which are required for post translation modification; cleavage sites in the encoded polypeptide; motifs in the encoded polypeptide (e.g. epitopes). Leader or other targeting sequences (e.g. hydrophobic anchoring regions) may be added or removed from the expressed protein to determine its location following expression.

Other desirable mutations may be made by random or site directed mutagenesis of the nucleic acid encoding the polypeptide in order to alter the activity (e.g. specificity) or stability of the encoded polypeptide.

Changes may be by way of conservative variation, i.e. substitution of one hydrophobic residue such as isoleucine, valine, leucine or methionine for another, or the substitution of one polar residue for another, such as arginine for lysine, glutamic for aspartic acid, or glutamine for asparagine. As is well known to those skilled in the art, altering the primary structure of a polypeptide by a conservative substitution may not significantly alter the activity of that peptide because the side-chain of the amino acid which is inserted into the sequence may be able to form similar bonds and contacts as the side chain of the amino acid which has been substituted out. This is so even when the substitution is in a region which is critical in determining the peptides conformation.

Also included are variants having non-conservative substitutions. As is well known to those skilled in the art, substitutions to regions of a peptide which are not critical in determining its conformation may not greatly affect its ability to raise antibodies because they do not greatly alter the peptide's three dimensional structure.

In regions which are critical in determining the peptides conformation or activity such changes may confer advantageous properties on the polypeptide. Indeed, changes such as those described above may confer slightly advantageous properties on the peptide e.g. altered stability or immunogenicity.

The invention will now be further described with reference to the following non-limiting Examples and Annexes. Other embodiments will occur to those skilled in the art in the light of these.

### Figures, Tables

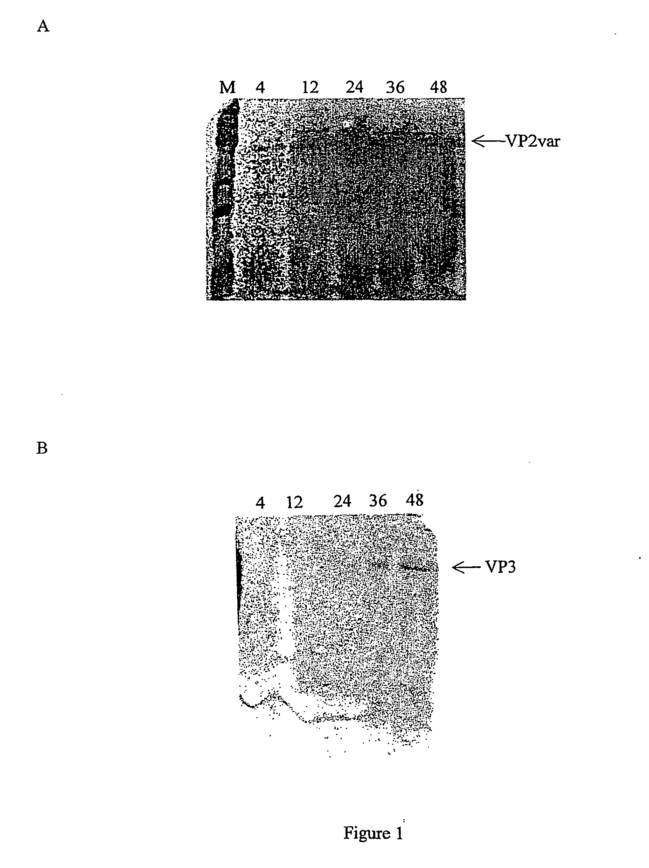

Figure 1 shows 10 % SDS-PAGE gels stained with Coomassie blue.
   (A) Shows samples of culture supernatant from *Pichia pastoris* expressing VP2var taken at 4, 12, 24, 36 and 48h following induction.
   (B) Shows samples of culture supernatant expressing VP3 taken at 4, 12, 24, 36 and 48h following induction.
Figure 2 shows the percentage of challenged fish whose antibodies bound to immobilized IPNV virus in ELISA tests.
Figure 3 shows the percentage of boosted fish whose antibodies bound to immobilized IPNV virus in ELISA tests.
Figure 4 shows the percentage of untreated fish whose antibodies bound to immobilized IPNV virus in ELISA tests.

### Sequence Annex

Annexes A-E show preferred IPNV-derived peptides of the invention.

### Examples

### Example 1 - Production of vp2var \ vp3 bivalent vaccine

### a)Isolation of IPNV coding regions

Plasmids containing the coding regions for VP2 and VP3 proteins from IPNV strain Sp were obtained from Dr David Smail, University of Aberdeen as glycerol stocks of transformed E. coli DH5α, from whom they are available on request.

In these stocks, the whole coding region of VP2 was cloned into the plasmid vector pUC18 and the whole coding region of VP3 was cloned into TA cloning vector (Stead., The application of recombinant DNA technology to the study of the infectious pancreatic necrosis virus. PhD Thesis, University of Aberdeen, 1994).

Plasmid was prepared from these glycerol stocks using the 3'-5' plasmid preparation kit under the manufacturer's instructions. PCR primers were designed to allow the amplification of a shortened coding region of VP2 and the whole coding region of VP3, while introducing enzyme restriction sites to allow the subsequent cloning into the *Pichia pastoris* expression vector, pPICZαB. The primers are shown below. The start codons for each protein are shown in the forward primers in bold and the restriction enzyme site are indicated by underlining. 
559                                    586
CTA ACA ACG GAA TTC ATG GAC AAA GTC   VP2var forward primer
            EcoRI site
 or in other embodiments:
5' gaagctgcagaggacaaagtcaac3' VP2var forward along with:

CGT TGC CGA TTG GCG GCC GCT GGT TGA TC   VP2var reverse primer
NotI site

2305                              2330
CCT GGG ACT GCA GAT GGC ATC AAA TG    VP3 forward primer
          PstI site

GTT ACA CCG CGG CCG CGT CTC CGC TGG G   VP3 reverse primer
NotI site

PCR was carried out using Ing of purified plasmid DNA containing the coding regions for VP2 and VP3, 25pmoles each PCR primer in 45µl of PCR master mix containing 2.5mM MgCl₂ (Advanced Biotechnologies). Cycling was carried out in a Perkin Elmer Thermocycler using the following cycling parameters; 94°C for 5 min followed by 35 cycles of 30s at 48°C, 1min 20s at 72°C, 30s at 94°C and a final incubation of 10min at 72°C. A 10µl aliquot of the resultant PCR reactions were electrophoretically separated through a 1.5% agarose gel containing 0.5µg/ml ethidium bromide. If amplification of the IPNV coding regions was successful, the DNA from the remainder of each PCR reaction was purified using the PCR clean up kit from Promega under manufacturer's instructions.

### b) Preparation of recombinant pPICZαB plasmids containing IPNV coding regions.

The purified IPNV PCR products underwent restriction enzyme digestion to facilitate cloning into the *Pichia pastoris* expression vector pPICZαB. VP2var PCR products were digested using the restriction endonucleases *Eco*RI and *Not*I or *Pst*I and *Not*I depending on the primer used, while the VP3 PCR products were digested using *Pst*I and *Not*I. Restriction digestions were set up by combining the following components; 30µl purified PCR product, 4µl restriction enzyme buffer, 4µl acetylated BSA (1mg/ml) and 1µl of each restriciton enzyme. The digestions were incubated at 37°C for 90min. In addition, pPICZαB plasmid was also digested using the same enzymes to allow each IPNV PCR product to be cloned. Plasmid restriction digestions were set up by combining the following components; 1µg plasmid DNA, 1µl restriction enzyme buffer, 1µl acetylated BSA(1mg/ml), 1µl each restriction enzyme and 5µl distilled water. The digestions were incubated at 37°C for 90 min. Following the incubation period, the digested DNA was purified from each sample by phenol/chloroform extraction and ethanol precipitation at -80°C for 20 min. The DNA was pelleted by centrifugation at 13,000rpm for 15 min, the pellets air dried and resuspended in 10µl distilled water. Ligations were prepared by combining the following components; 5µl digested IPNV PCR product, 1µl digested pPICZαB plasmid, 1µl 10x ligase buffer, 2µl distilled water and 1µl T4 DNA ligase. The ligations were incubated overnight at 4°C.

The following day, a 3µl aliquot of each ligation mix was used to transform electrocompetent *E. coli* TOP10F' cells (Invitrogen) under manufacturer's instructions. Following cell recovery, aliquots of the transformed cells were plated onto LB agar plates containing 25µg/ml zeocin and the plates incubated, inverted, overnight at 37°C. Each resultant colony was used to inoculate 5ml of LB medium containing 25µg/ml zeocin which was subsequently incubated overnight at 37°C with vigorous aeration. Recombinant pPICZαB plasmid was prepared from 1ml of each overnight culture using the 3'-5' plasmid preparation kit. The remaining culture was used to prepare glycerol stocks which were stored at -80°C. Recombinant plasmids were screened for the presence of a insert by restriction digestion with either *Eco*RI and *Not*I (VP2var) or *Pst*I and *Not*I (VP3) using the protocol outlined previously. Digestions were electrophoretically separated through a 1.5% agarose gel containing 0.5µg/ml ethidium bromide. Plasmids which contained the correct sized inserts were further analysed by automated DNA sequencing.

### c) Preparation of Pichia pastoris cell lines expressing VP2var and VP3

Recombinant pPICZαB plasmids which had been shown to contain either the coding region for VP2var or VP3 by restriction digestion analysis and DNA sequence analysis were prepared for transformation into *Pichia pastoris* strain GS115 as outlined below. Large scale plasmid preparations of each recombinant pPICZαB plasmid were carried out using the 3'-5' plasmid prepartion kit under manufacturer's instuctions. Approximately 10µg of each plasmid was prepared. The recombinant plasmids were linearised prior to their transformation into *P. pastoris* GS115 using the restriction enzyme *Sac*I by combining the following components; 5µg recombinant plasmid (40µl approximately), 6µl 10x restriction enzyme buffer, 6µl acetylated BSA(1mg/ml), 6µl distilled water and 2µl SacI. The digestions were incubated at 37°C for 90 min. Following this incubation the digested plasmid were purified by phenol/chloroform extraction and ethanol precipitation at -80°C for 20 min. The linearised plasmids were resuspended in 10µl distilled water.

Electrocompetent *Pichia pastoris* GS115 cells were prepared as outlined below. A single colony of GS115 was used to inoculate 5ml of YPD medium which was then incubated overnight at 30°C with vigorous aeration. The following day 0.5ml of this overnight culture was used to inoculate 500ml of fresh YPD medium which was grown overnight as before. The cells were pelleted at 1500g for 5 min at 4°C and the pellet resuspended in 500ml of ice-cold sterile, distilled water. The cells were pelleted as before and resuspended in 250ml of ice-cold sterile, distilled water. The cells were pelleted again and resuspended in 20ml of ice-cold sterile 1M sorbitol, pelleted for the last time and resuspended in 1ml of ice-cold 1M sorbitol. The cells were used immediately. 80µl of the electrocompetent cells were mixed with 10µl linearised plasmid described above, and transferred to an ice-cold 0.2cm electroporation cuvette. The cuvette was incubated on ice for 5min. The cell and DNA mix was pulsed in a Bio-Rad Gene Pulser with a charging voltage of 1500V, a capacitance of 25µF and a resistance of 200Ω. 1ml of ice-cold 1M sorbitol was added and the contents transferred to a sterile 15ml tube. The cells were allowed to recover by incubation at 30°C for 1-2h without shaking. Aliquots of the cells were plated onto YPDS plates containing 100µg/ml zeocin. The plates were incubated for 2-3 days at 30°C until colonies formed. The resultant colonies were screened for the Mut Phenotype as outlined below.

Colonies resulting from the transformation of *Pichia pastoris* GS115 transformation were replica plated onto MMH and MDH plates. The plates were incubated at 30°C for 2-3 day until colonies formed. Colonies which are Mut⁺ show normal growth on both plates while colonies which are Mut^{s} show normal growth on MDH plates but little is any growth on MMH plates. This method revealed all of the cell lines containing VP2var coding region to be Mut⁺ while all of the cell lines containing VP3 coding region to be Mut^{s}.

Small scale expressions were carried out using the recombinant *Pichia pastoris* GS115 colonies in order to screen for expression of VP2 var and VP3 in these cell lines.

*VP2var containing Pichia (Mut⁺):* Each colony was used to inoculate 25ml of BMGY medium in a 250ml flask. The cultures were incubated overnight at 30°C with vigorous aeration. The following day the cells were harvested at 1500g for 5 min at room temperature and the cell pellet resuspended in 100ml of BMMY to induce protein expression. The culture was incubated in a 11 flask at 30°C with vigorous aeration with 100% methanol being added to a final concentration of 0.5% every 24h. 1ml aliquots were removed at the following time points; 0, 6, 8, 24, 32, 48, 56, 72 and 80 hours, the cells were pelleted and the supernatant transferred to a fresh tube. Both cell pellet and supernatant were stored at -80°C until all time point samples had been collected.

*VP3 containing Pichia (Mut^{s}):* Each colony was used to inoculate 100ml of BMGY medium in a 11 flask. The cultures were incubated overnight at 30°C with vigorous aeration. The following day the cells were harvested at 1500g for 5 min at room temperature and the cell pellet resuspended in 20ml of BMMY to induce protein expression. The culture was incubated in a 250ml flask at 30°C with vigorous aeration with 100% methanol being added to a final concentration of 0.5% every 24h. 1ml aliquots were removed at the following time points; 0, 24, 48, 72 and 96 hours, the cells were pelleted and the supernatant transferred to a fresh tube. Both cell pellet and supernatant were stored at -80°C until all time point samples had been collected.

The samples collected during the small scale expression were analysed by SDS-PAGE through a 15% acrylamide gel. Two identical gels were run for each sample, one of which was stained with Coomassie Brilliant blue to visualise the proteins and the other was western blotted onto nitrocellulose and immunoblotted with specific anti-VP2 and ant-VP3 monoclonal antibodies. This allowed the optimum expression period to be determined for each cell line.

Glycerol stocks were prepared for each cell line which showed good expression of VP2var or VP3. These were stored at -80°C.

### Example 2 - Alternative production of vp2var \ vp3

### a) Construction of recombinant plasmids

PCR was used to amplify the coding regions of VP3 or VP2var using the PCR primers:
VP3 forward 2342-2367
   5' gacgctgcagtgcaacgcctcctg 3'
VP3reverse 2936-2967
   5' gtgcagcggccgccgggggtcgtcgtttcatc 3'
VP2var forward 602-630
   5' accactgcagtcacagtcctgaatc3'
VP2var reverse 1143-1172
   5' gagcgcggccgccgcaattccgttccctg3'

The PCR products were digested using the restriction enzymes *PstI* and *NotI* to produce cohesive ends. The sequences were ligated into the expression plasmid pPICZαB which had been digested using the restriction enzymes *PstI* and *NotI* and dephosphorylated using calf intestinal alkaline phosphatase. The recombinant plasmids were transformed into the E. coli strain TOP10F' using standard electroporation. The transformant mix was plated out onto LB agar plates containing 25µg/ml zeocin. Transformants were used to inoculate 2ml LB medium containing 25µg/ml zeocin and were grown overnight at 37°C with shaking. Plasmid was isolated from each overnight culture using standard methodology and the plasmids DNA sequenced to confirm the sequence of the inserts.

### b) Generation of recombinant Pichia pastoris clones

5-10µg of recombinant plasmid DNA was digested with *PmeI* using standard methodology. 20µl of the digest mix was used to transform 100µl competent *Pichia pastoris* strain GS115 cells using the Easycomp Transformation kit (Invitrogen) under manufacturer's instructions. The transformation mix was plated out onto YPD agar plates containing 100µg/ml zeocin and incubated at 30°C for 2-4 days.

### c) Expression of recombinant VP3 or VP2var protein

A single colony of recombinant Pichia containing pPICZαB/VP3 or pPICZαB/VP2var was used to inoculate 25ml BMGH medium in a 250ml baffled conical flask. The culture was grown at 30°C in a shaking incubator (250-300rpm) until the culture reached an OD₆₀₀ of 3.0. The cells were harvested by centrifugation at 2000rpm for 5 min at room temperature. The supernatant was decanted and the pellet resuspended in BMMH medium to an OD₆₀₀ of 1.0. The culture was placed in a litre baffled conical flask and incubation continued at 30°C in a shaking incubator (250-300rpm) for a period of 72-108h. 100% methanol was added to each culture, every 24h, to a final concentration of 0.5%. Following expression the recombinant protein was harvested by centrifugation at 2000rpm for 10min at room temperature. The supernatant was decanted, filter sterilise and aliquoted into 1ml samples. The supernatant samples were stored at -20°C. A sample of each expression culture was analysed for protein expression using SDS-PAGE and western blotting using specific antisera. The sequences are given in Annex C and D.

### Example 3 - production of vp2var \ vp3 hybrid clone

### a) Construction of recombinant plasmids

PCR was used to amplify the coding regions of VP3 and VP2var using the PCR primers:
VP3 forward 2342-2367
   5' gacgctgcagtgcaacgcctcctg 3'
VP3 reverse 3023-3044
   5' ctctctagagtctccgctggg 3'
VP2var forward 603-622
   5' ccctcagagtcacagtcctg 3'
VP2var reverse 1143-1172
   5' gagcgcggccgccgcaattccgttccctg3'

The amplified nucleotide sequences, when ligated together, code for a hybrid protein consisting of amino acids 9-244 of VP3 fused to amino acids 163-357 of VP2.

The PCR products were mixed together anddigested using the restriction enzymes *PstI, XbaI* and *NotI* to produce cohesive ends. The VP3 and VP2var PCR prodcuts were ligated together to produce a hybrid sequence. The hybrid sequence was subsequently ligated into the expression plasmid pPICZαB which had been digested using the restriction enzymes *PstI* and *NotI* and dephosphorylated using calf intestinal alkaline phosphatase. The recombinant plasmids were transformed into the E. coli strain TOP10F' using standard electroporation. The transformant mix was plated out onto LB agar plates containing 25µg/ml zeocin. Transformants were used to inoculate 2ml LB medium containing 25µg/ml zeocin and were grown overnight at 37°C with shaking. Plasmid was isolated from each overnight culture using standard methodology and the plasmids DNA sequenced to confirm the sequence of the inserts.

### b) Generation of recombinant Pichia pastoris clones

5-10µg of recombinant plasmid DNA was digested with *PmeI* using standard methodology. 20µl of the digest mix was used to transform 100µl competent *Pichia pastoris* strain GS115 cells using the Easycomp Transformation kit (Invitrogen) under manufacturer's instructions. The transformation mix was plated out onto YPD agar plates containing 100µg/ml zeocin and incubated at 30°C for 2-4 days.

### c) Expression of recombinant VP3 or VP2var protein

A single colony of recombinant Pichia containing pPICZaB/VP3VP2var hybrid was used to inoculate 25ml BMGH medium in a 250ml baffled conical flask. The culture was grown at 30°C in a shaking incubator (250-300rpm) until the culture reached an OD₆₀₀ of 3.0. The cells were harvested by centrifugation at 2000rpm for 5 min at room temperature. The supernatant was decanted and the pellet resuspended in BMMH medium to an OD₆₀₀ of 1.0. The culture was placed in a litre baffled conical flask and incubation continued at 30°C in a shaking incubator (250-300rpm) for a period of 72-108h. 100% methanol was added to each culture, every 24h, to a final concentration of 0.5%. Following expression the recombinant protein was harvested by centrifugation at 2000rpm for 10min at room temperature. The supernatant was decanted, filter sterilise and aliquoted into 1ml samples. The supernatant samples were stored at -20°C. A sample of each expression culture was analysed for protein expression using SDS-PAGE and western blotting using specific antisera.

### Example 4 - Large scale expression of VP2var and VP3 for immunisation

Antigens as described above for use in the vaccines of the present invention may optionally be prepared as follows.
i) *For Mut⁺ secreted expression (GS115*/ *pPICZ*α*B*/*VP2var*/*Mut⁺) :* A single colony was used to inoculate 25ml of BMGY medium in a 250ml baffled flask. The culture was incubated at 28-30°C (250-300rpm) until the culture reached an OD₆₀₀ = 2-6 (approximately 16-18h). The overnight culture was used to inoculate 11 of BMGY in a 3 or 41 baffled flask and was grown at 28-30°C with vigorous shaking (250-300rpm) until the culture reaches an OD₆₀₀ = 2-6. The cells were harvested by centrifugation at 1500-3000 xg for 5 min at room temperature. Expression was induced by resuspending the pellet to an OD₆₀₀ = 1.0 (2-6 litres) in BMMY medium to start induction. The cultures were grown at 28-30 °C with shaking. 100% methanol was added to 0.5% every 24h until the optimum time of induction was reached. For GS115/ pPICZαB/VP2var/Mut⁺ 1 the optimum time is 56h; for GS115/ pPICZαB /VP2var/Mut⁺ 40 the optimum time is 32h; for GS115/ pPICZαB /VP2var/Mut⁺ 34 the optimum time is 24h. The cells were harvested by centrifugation at 1500x g for 5 min at room temperature. The supernatant was saved, chilled to +4 °C and filter sterilised. The expressed protein was stored in 1ml aliquots at -80 °C.
*ii) For Mut^{s} secreted expression (GS115*/ *pPICZ*α*B*/*VP3*/*Mut^{s}):* A single colony was used to inoculate 10ml of BMGY medium in a 100ml baffled flask. This was grown at 28-30°C (250-300rpm) until the culture reached an OD₆₀₀ = 2-6 (approximately 16-18h). This overnight culture was used to inoculate 11 of BMGY in a 3 or 41 baffled flask and was grown at 28-30°C with vigorous shaking (250-300rpm) until the culture reaches an OD₆₀₀ = 2-6. The cells were harvested by centrifugation at 1500-3000 xg for 5 min at room temperature. Expression was induced by resuspending the cell pellet in 1/5 to 1/10 of the original culture volume in BMMY medium (approximately 100-200ml). The culture was placed in a 1litre baffled flask and returned to incubator at 28-30°C with shaking. 100% methanol was added to 0.5% every 24h until the optimum time of induction was reached. For GS115/ pPICZαB/VP3/Mut^{s} 30.16 the optimum time is 72h; for GS115/ pPICZαB/VP3/Mut^{s} 30.17 the optimum time is 48h; for GS115/ pPICZαB/VP3/Mut^{s} 30.18 the optimum time is 48h; for GS115/ pPICZαB/VP3/Mut^{s} 28 the optimum time is 72h. The cells were harvested by centrifugation at 1500-3000 x g for 5 min at room temperature. The supernatant was saved, chilled to +4 °C and filter sterilised. The expressed protein was stored in 1ml aliquots at -80 °C.

A sample of each filter sterilised recombinant protein was run through a 10% SDS-PAGE and Coomassie stained to check the induction.

### Example 5- IPN dose response in salmon

Atlantic salmon, *Salmo salar,* used in these experiments were reared at the Fish Cultivation Unit of the Marine Laboratory, Aultbea, Wester Ross, Scotland. Prior to all experimental procedures the fish were anaesthetised using ethyl-4-amino benzoate (Benzocaine, BDH Chemicals, Poole, Dorset, UK).

All experiments were carried out in one metre tanks containing 350 litres of fresh water, supplied with ca 10 litres per minute per tank. Fish were fed (Mainstream diets, BP Nutrition) daily to satiation.

### Immunisation.

Five doses were used: 10, 35, 70, 100 and 150 µg of each VP2var and VP3 combined. The bivalent vaccine was diluted in PBS and mixed with the adjuvant (Montanide ISA 711®, Sepic) at a ratio of 30:70, respectively. Fish were i.p. injected with 0.1 ml vaccine containing 20, 70, 140, 200 and 300 µg bivalent vaccine. Fish i.p. injected with PBS plus adjuvant (ratio 30:70) were used as control. During the experimental period fish were kept at 7°C for 4 weeks and then transferred to 14°C.

Groups are shown in Table 1:

**Table 1**

| Dose per fish | No. Fish |
|---|---|
| Control | 60 |
| 20 µg | 59 |
| 70 µg | 59 |
| 140 µg | 45 |
| 200 µg | 55 |
| 300 µg | 50 |

### Challenge.

Before challenge 10 fish of each group were bled (except 140 µg/fish dose where 5 fish were bled). Eleven weeks post-vaccination fish were split in three groups:
1. One group of fish were intraperitoneally challenged with IPNV grown on CHSE-214 cells at a dose of 1.7 x 10⁷ TCID₅₀ per fish. Blood samples were taken 4 and 10 weeks post-challenge.
2. The second group were administrated a two weeks course of oral boost vaccine. Blood samples were taken 4 and 10 weeks after the oral boost was finished.
3. The third group of fish was left untreated. Samples were taken 18 and 24 weeks post-vaccination.

### Example 6 - IPNV enzyme-linked immunobsorbant assay (ELISA)

Fish were immunised with bivalent vaccine (VP2var and VP3) as described in Example 5.

IPNV PEG precipitated virus is diluted with 0.05 M carbonate-bicarbonate buffer, pH 9.6, to give 5 x 10⁷TCID₅₀/ml and used (100 µl) to coat individual wells (Immulon 4 HBX, Dynex Technologies Inc, USA). The coated plates were incubated at 4°C for 48 h, washed in phosphate-buffered saline (PBS) containing 0.05% Tween-20 (PBS-Tween), blocked with 5% non fat dry milk in PBS-Tween for 1 h at 37°C, washed with PBS-Tween and stored at -80°C until used.

All subsequent dilutions and washing between incubations were carried out in PBS-Tween. Salmon antisera were serially two-fold diluted (1:60 to 1:1920) and incubated in duplicate at 4°C overnight. A pool of sera from control fish was used as a negative control. A positive control was used in all plates. Incubation with PBS-Tween was used as a blank. After washing, Mouse anti-salmon Ig (4C10) diluted 1:8 was incubated for 2 h at room temperature. Horseradish peroxidase conjugate goat anti-mouse IgG (Sigma) diluted 1:1000 was incubated 1 h at room temperature.

Tetramethylbenzidine (TMB, Sigma), 100 µl/well, was added as substrate and incubated for 30 min at room temperature. Plates were read spectrophotometrically at 630 nm using an ELISA reader (DIAS, Dynatech Laboratories).

### Example 7 - IPNV serum neutralisation assay

The protective effect of vaccination with the IPNV subunit vaccine was verified using a neutralising antibody assay, as follows.

Fish were immunized with bivalent vaccine (VP2var and VP3) as described in Example 5.

Heat-inactivated serum samples from vaccinated fish were prepared by serial dilution in E-MEM + 2% foetal bovine serum (FBS) and mixed with live IPN virus in 96-well microplates to a final concentration of 500 TCID₅₀ per 100 µl well. Following incubation for 1 h at room temperature, 50 µl aliquots of these samples containing virus and plasma at appropriate concentrations were applied to wells containing confluent chinook salmon embryo (CHSE-214) cells in 75 µl E-MEM +10 % FBS. Controls were prepared by substitution of pooled normal salmon serum or omission of virus as appropriate. All cultures were incubated for 7 days at 15 °C, and then virus induced cell lysis was determined by measurement of absorbance on the microplate-format spectrophotometer.

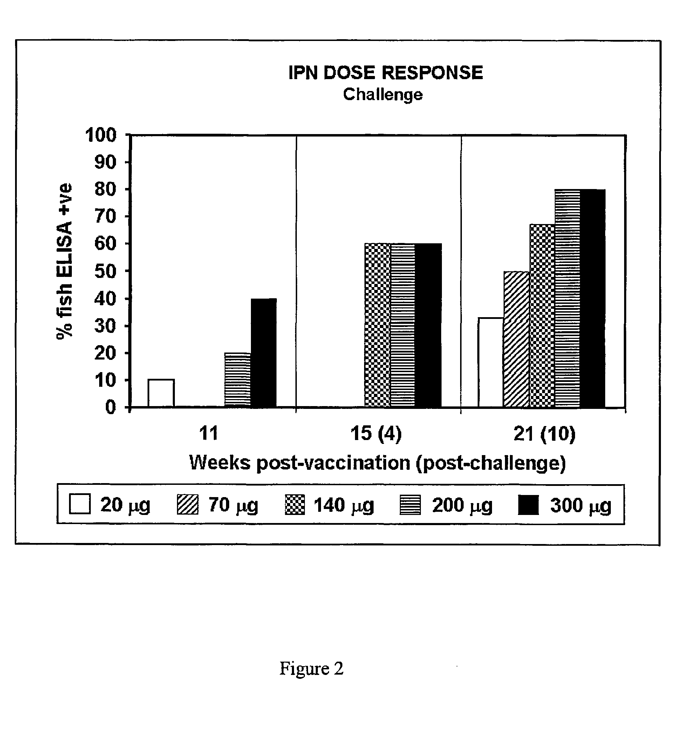

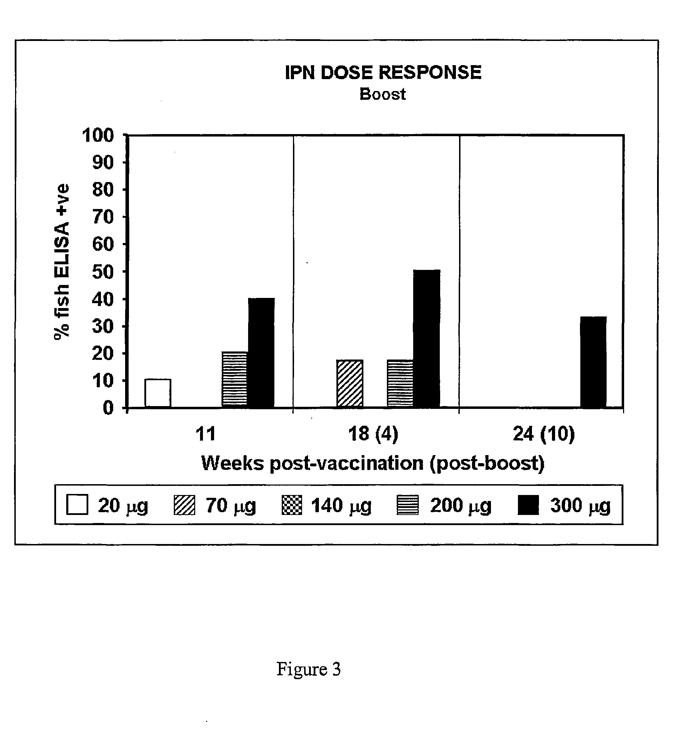

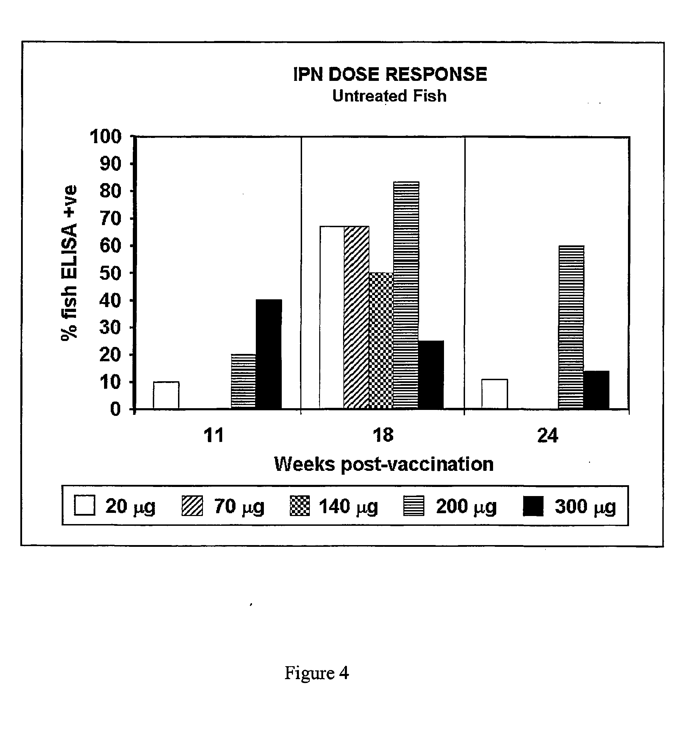

The efficacy of the vaccine was demonstrated by the number of fish showing increased titres of antibody and neutralising antibodies (Figs 2-4, Tables 2-3). Table 2 is shown hereinafter.

**Table 3a**

| Group | No. in group | Treatment |
|---|---|---|
| 1 | 60 fish | Control @ 100 µl/fish |
| 2 | 59 fish | 20 µg @ 100 µl/fish |
| 3 | 59 fish | 70 µg @ 100 µl/fish |
| 4 | 45 fish | 140 µg @ 100 µl/fish |

**Table 3b**

| Group | No. of fish assayed | No. with neutralising Abs |
|---|---|---|
| 2 | 3 | 2/3 |
| 3 | 4 | 3/4 |
| 3 | 5 | 3/3 |

Table 3 shows the number of fish whose antibodies neutralized IPNV in a neutralization assay. (3a) shows the details of the fish groups. The fish used were those who gave positive results in the ELISA tests. (3b) shows the results of the neutralization assays. The serum was taken from unchallenged fish 18 weeks after vaccination.

### Example 8 - further vaccine trials

The above data were confirmed in further trials. These trials used the following:
(1) An injection vaccine containing the two IPN proteins described above, an inactivant, a diluent, and the synthetic oil adjuvant Montanide ISA711 (AquaVac™ IPN - for injection)
(2) An oral (booster) containing the two IPN proteins, a diluent, and an adjuvant carrier system which includes an oil and an emulsifier (AquaVac™ IPN Oral Vaccine).
(3) A multivalent injection vaccine containing the two IPN proteins, *Aeromonas salmonicida* antigens, an inactivant, a diluent, and the synthetic oil adjuvant Montanide ISA711 (AquaVac™ FNM ^{PLUS} IPN Vaccine for injection).
(4) A multivalent containing the IPN proteins, *Aeromonas salmonicida* and one or more Vibrio (including *V.anguillarum, V. salmonicida* and *V. viscosus)* antigens), an inactivant, a diluent, plus the synthetic oil adjuvant Montanide ISA711. (AquaVac™ FV4-IPN for injection).

The results are shown in Table 4 hereinafter.

As can be seen from the Table, the vaccines according to the present invention, as produced in *Pichia pastoris,* were surprisingly efficacious. Considering Ref10, the vaccine showed considerably higher efficacy than Norvax(R)Compact 6 VAT (Intervet Norbio) which is based on rVP2 produced in *E. coli.*

In related comparative studies, a VP2/VP3 vaccine produced in *Pichia* resulted in a greater proportion on fish which produced antibodies specific for IPNV than a comparable *E.coli* vaccine. Additionally, fish immunized with the *Pichia*-produced vaccine, and then challenged with IPN virus were able to clear the virus from their systems. This was not the case for the *E.coli* produced vaccine.

### References

Wolf, K., Snieszko, S.F., Dunbar, C.E. & Pyle, E. (1960). Virus nature of infectious pancreatic necrosis in trout. Proceedings of Society for Experimental Biology and Medicine, 104; 105-108.
Pilcher. K.S. & Fryer, J.L. (1980). The viral disease of fish: a review through 1978. Critical Reviews in Microbiology, 7: 287-364.
Park, J.W. (1991). Characteristics of Infectious Pancreatic Necrosis Virus isolated from rainbow trout in Korea, Ph.D. Thesis, Seoul National University.
Cohen, J., Poinsard, A. & Scherrer, R. (1973). Physiochemical and morphological features of infectious pancreatic necrosis virus. Journal of General Virology, 21: 485-98.
Dobos, P. (1977). Virus specific protein synthesis in cells infected with infectious pancreatic necrosis virus. Journal of Virology, 21: 242-258.
Chang, N., MacDonald, R.D. & Yamamoto, T. (1978). Purification of infectious pancreatic necrosis (IPN) virus and comparison of polypeptide composition of different isolates. Canadian Journal of Microbiology, 24: 19-27.
MacDonald, R.D. & Dobos, P. (1981). Identification of proteins encoded by each genomic segment of infectious pancreatic necrosis virus. Virology, 114: 414-422.
Persson, R.H. & MacDonald, R.D. (1982). Evidence that infectious pancreatic necrosis virus has a genome linked protein. Journal of Virology, 44: 437-443.
Dobos, P., Hill, B.J., Hallett, R., Kells, D.T.C., Becht, H. & Teninges, D. (1979). Biophysical and biochemical characterisation of five animal viruses with bisegmented double-stranded RNA genomes. Journal of Virology, 32: 593-605.
Dobos, P. & Rowe, D. (1977). Peptide map comparison of infectious pancreatic necrosis virus- specific polypeptides. Journal of Virology, 24: 805-820.

### Sequence Annex

Annex A - VP2 var amino acids 154-326 
QDKVNNQ LVTKGVTVLN LPTGFDKPYV RLEDETPQGL QSMNGAKMRC
TAAIAPRRYE IDLPSQRLPP VPATGTLTTL YEGNADIVNS TTVTGDINFS
LAEQPANETK FDFQLDFMGL DNDVPVVTVV SSVLATNDNY RGVSAKMTQS
IPTENITKPI TRVKLSYKIN Q
Annex B - VP3 amino acids 1-238 
MASNASGMDE ELQRLLNATM ARAKEVQDAE IYKLLKLMAW TRKNDLTDHM
YEWSKEDPDA LKFGKLISTP PKHPEKPKGP DQHHAQEARA TRISLDAVRA
GADFATPEWV ALNNYRGPSP GQFKYYLITG REPEPGDEYE DYIKQPIVKP
TDMNKIRRLA NSVYGLPHQE PAPEEFYDAV AAVFAQNGGR GPDQDQMQDL
RELARQMKRR PRNADAPRRT RAPAEPAPPG RSRFTPSG
Annex C - VP2var amino acids 167-352 
TVLNLP TGFDKPYVRL EDETPQGLQS MNGAKMRCTA AIAPRRYEID
LPSQRLPPVP ATGTLTTLYE GNADIVNSTT VTGDINFSLA EQPANETKFD
FQLDFMGLDN DVPVVTVVSS VLATNDNYRG VSAKMTQSIP TENITKPITR
VKLSYKINQQ TAIGNVATLG TMGPASVSFS SGNGN
Annex D - VP3 amino acids 12-199 
QRLLNA TMARAKEVQD AEIYKLLKLM AWTRKNDLTD HMYEWSKEDP
DALKFGKLIS TPPKHPEKPK GPDQHHAQEA RATRISLDAV RAGADFATPE
WVALNNYRGP SPGQFKYYLI TGREPEPGDE YEDYIKQPIV KPTDMNKIRR
LANSVYGLPH QEPAPEEFYD AVAAVFAQNG GRGPDQDQMQ DLRELARQMK
RRP
Annex E - amino acids 12-238(VP30 +163-352(VP2var)

QRLLNA TMARAKEVQD AEIYKLLKLM AWTRKNDLTD HMYEWSKEDP

DALKFGKLIS TPPKHPEKPK GPDQHHAQEA RATRISLDAV RAGADFATPE
WVALNNYRGP SPGQFKYYLI TGREPEPGDE YEDYIKQPIV KPTDMNKIRR
LANSVYGLPH QEPAPEEFYD AVAAVFAQNG GRGPDQDQMQ DLRELARQMK
RRPRNADAPR RTRAPAEPAP PGRSRFTPSG DSTVTVLNLP TGFDKPYVRL
EDETPQGLQS MNGAKMRCTA AIAPRRYEID LPSQRLPPVP ATGTLTTLYE
GNADIVNSTT VTGDINFSLA EQPANETKFD FQLDFMGLDN DVPVVTVVSS
VLATNDNYRG VSAKMTQSIP TENITKPITR VKLSYKINQQ TAIGNVATLG
TMGPASVSFS SGNGN

**Table 2 shows the number of challenged, boosted and untreated fish whose antibodies bound to immobilized IPNV in ELISA tests.**

| | **Prechallenge** | **IPNV CHALLENGE** | | **BOOST COURSE** | | **UNTREATED FISH** | |
|---|---|---|---|---|---|---|---|
| | wks post-vaccination | wks post-vaccination (post-challenge) | | wks post-vaccination (post-boost) | | wks post-vaccination | |
| | 11 | 15(4) | 21(10) | 18(4) | 24(10) | 18 | 24 |
| DOSE | ELISA+ve* | ELISA+ve | ELISA+ve | ELISA+ve | ELISA+ve | ELISA+ve | ELISA+ve |
| Contr ol | (1:300) | (1:160) | (1:45) | (1:192) | (1:192) | (1:120) | (1:120) |
| 20µg | 1/10 | 0/10 | 3/9 | 0/6 | 0/9 | 4/6 | 1/9 |
| 70µg | (1:240) | 0/10 | (1:120) | 1/6 | 0/8 | (1:150) | (1:120) |
| 140µg | 0/10 | 6/10 | 5/10 | (1:240) | 0/4 | 4/6 | 0/9 |
| 200µg | (1:720) | (1:167) | (1:156) | 0/6 | 0/5 | (1:180) | 0/3 |
| 300µg | 0/5 | 6/10 | 6/9 - | 1/6 | 2/6 | 3/6 | 3/5 |
| | 2/10 | (1:160) | (1:200) | (1:240) | (1:600) | (1:100) | (1:120) |
| | (1:720) | 6/10 | 8/10 | 3/6 | | 5/6 | 1/7 |
| | 4/10 | (1:260) | (1:231) | (1:320) | | (1:168) | (1:120) |
| | (1:900) | | 8/10 | | | 1/4 | |
| | | | (1:203) | | | (1:960) | |

| | | | | | | | |
|---|---|---|---|---|---|---|---|
| *No. fish ELISA+ve/No. fish sampled (mean ELISA titre from positive fish) | | | | | | | |

**TABLE 4 - EFFICACY DATA FOR IPN VACCINES**

| Ref | Fish numbers | Study Location | Vaccine used (ref) /Route of administration | *Efficacy results* |
|---|---|---|---|---|
| 1 | 2000 | Shetlands | AquaVac FNM Plus IPN / by injection (3) | Fish were vaccinated in the hatchery 10 weeks prior to transfer to sea water. A natural infection of IPN occurred about 11 weeks after transfer to sea water. |
| | | | | • 5% of unvaccinated fish died. |
| | | | | • 3.2% of vaccinated fish died |
| | | | | • RPS = 35% |
| 2 | ? | Marine Harvest Lab challenge test | AquaVac FNM Plus IPN / by injection (3) | Fish were given 0.1 ml injection of vaccine, then transferred to sea water after 5 months, then challenged by cohabitation with IPN infected fish 4 weeks after transfer. By 14 days post challenge, |
| | | | | • 40% of unvaccinated controls had died |
| | | | | • 15% of vaccinates had died |
| | | | | RPS (relative percent survival) = 63% |
| 3 | 126,000 vaccinates | Chile | • IPN/ by injection (1) | Fish injection vaccinated in February Transferred to sea water in June |
| | 27,500 controls | (Marine Harvest) | | Oral booster vaccine given 2 weeks later. |
| | | | • IPN Oral (2) booster dose | A natural infection of IPN occurred about 8 weeks after transfer to sea water. In first 4 weeks after infection started, |
| | | | | • 6% of unvaccinated controls died |
| | | | | No IPN deaths in vaccinated fish. |
| 4 | 100,000 vaccinates | Chile | • IPN/ by injection (1) | Fish injection vaccinated in February. Transferred to sea water 15 weeks later. Oral booster given 11-13 weeks later |
| | | (Robinson Crusoe) | • *IpN Oral (2) booster dose* | . A natural infection of IPN occurred 14 weeks after transfer to sea water (2-4 weeks after IPN Oral booster vaccination.) Outbreak lasted 3 weeks. |
| | | | | • 2% of unvaccinated controls died in first week |
| | | | | • Accumulative mortality in controls over 4 week period was 4.6% |
| | | | | No IPN deaths in vaccinated fish |
| 5 | 82,000 vaccinates | UK | • AquaVac FNM Plus IPN / by injection | Fish injection vaccinated in February-March. Transferred to sea water. Oral booster given about 3 months after injection dose. |
| | 122,000 controls (FNM Plus) | (Papil) | (3) | A natural infection of IPN occurred about 4 weeks after administration of oral booster dose. |
| | | | • IpN Oral (2) booster | • Mean IPN deaths in controls = 5% |
| | | | | • Mean IPN deaths in vaccinates = 2.95% |
| | | | dose | RPS =42% |
| 6 | | Chile | • IPN/ by injection (1) | Mortalities at Site 1: |
| | | (Aqua Class) | | • Controls 9% |
| | | 2 sites | | • Vaccinates 3% |
| | | | • *IPN Oral* (2) booster dose | Mortalities at Site 2: |
| | | | | • Controls 4.2% |
| | | | | Vaccinates 1.5% |
| 7 | 44,000 vaccinates | Chile | • IPN/ by injection (1) | Mortalities at Site 1: |
| | | (Multi Export) | | • Controls 6.41% |
| | 115,000 controls | 2 sites | | • Vaccinates 1.39% RPS = 78% |
| | | | • *IPN Oral (2)* booster dose | Mortalities at Site 2: ( 2 groups of vaccinates, 1 of controls) |
| | | | | • Controls 3.51% |
| | | | | Vaccinates 0.98% and 0.75% RPS = 77% and 84% |
| 8 | | Marine Harvest | • FV4-IPN (4) by injection | Mortalities: |
| | | | | • Controls 14% |
| | | | | • Vaccinates 3% |
| | | | | RPS = 79% |
| 9 | | VIKAN (Norway) Challenge | • FV4-IPN (4) by injection Lab. | Mortalities: |
| | | | | • Controls 12% |
| | | | | • Vaccinates 7% |
| | | | | RPS = 42% |
| 10 | 90,000 vacc. with FV4-IPN + IPN Oral | Hydrotech (Norway) | • FV4-IPN (4) by injection | Mortalities: |
| | 90,000 with Compact 6 (*E. coli* expression system) | | | • Controls (Compact 6) 8.12% |
| | | | | • Vaccinates 2.79% |
| | | | • *IPN Oral (2)* booster dose | |

| | | | | |
|---|---|---|---|---|
| (1) AquaVac™ IPN - for injection (2) AquaVac™ IPN Oral (3) AquaVac™ FNM ^{PLUS} IPN -for injection (4) AquaVac™ FV4-IPN - for injection | | | | |

## Claims

1. A process for producing bivalent vaccine for use against infectious pancreatic necrosis virus (IPNV) in fish, **characterised in that** it comprises:
(i) culturing yeast host cells which express two different IPNV polypeptides which are respectively:
(a) an IPNV VP3 polypeptide or a fragment thereof comprising a sequence having at least 90% sequence identity with the sequence of Annex B or Annex D
(b) an IPNV VP2 polypeptide or a fragment thereof comprising a sequence having at least 90% sequence identity with the sequence of Annex A or Annex C,
wherein in each case the polypeptide or fragment is capable of stimulating the production of antibodies which bind IPNV,
and wherein the expressed IPNV polypeptides are secreted from the host cells into the culture supernatant,(ii) formulating the IPNV polypeptides as a vaccine.

2. A process as claimed in claim 1 wherein the yeast host cell is *Pichia pastoris.*

3. A process as claimed in claim 1 wherein host cells are separated from the supernatant, and the supernatant containing secreted IPNV polypeptides is formulated as the vaccine.

4. A process as claimed in claim 1 or claim 3 wherein the IPNV polypeptides are secreted with a signal sequence which is the yeast α mating factor signal sequence.

5. A process as claimed in any one of the preceding claims wherein the vaccine is formulated by dilution with PBS or addition of adjuvant or a combination of these.

6. A process as claimed in any one of claims 1 to 5 which comprises the steps of:
(i) isolating a nucleic acid IPNV coding region encoding:
(a) an IPNV VP3 polypeptide or a fragment thereof comprising a sequence having at least 90% sequence identity with the sequence of Annex B or Annex D, or
(b) an IPNV VP2 polypeptide or a fragment thereof comprising a sequence having at least 90% sequence identity with the sequence of Annex A or Annex C,
wherein in each case the polypeptide or fragment is capable of stimulating the production of antibodies which bind IPNV,(ii) preparing a recombinant DNA plasmid containing the IPNV coding region,
(iii) preparing yeast cell lines expressing the IPNV polypeptide,
(iv) screening for expression of the IPNV polypeptide in the cell lines.

7. A process as claimed in any one of claims 1 to 6 wherein the IPNV polypeptides are encoded by multiple copies of the coding region of IPNV genes ligated together to form a single open reading frame with a single initiation and termination codon to produce a multivalent IPNV antigen.

8. A process as claimed in any one of claims 1 to 6 wherein the IPNV polypeptides are expressed in different yeast host cells.

9. A process as claimed in any one of claims 1 to 8 wherein each IPNV polypeptide is present as two or more copies of antigen fused together in the correct orientation for expression as a single polypeptide such as to produce multimeric IPNV antigens.

10. A process as claimed in any one of claims 1 to 6 wherein the IPNV polypeptides are VP3 having the sequence of Annex B or Annex D, and VP2 having the sequence of Annex A or Annex C.

11. A process as claimed in claim 10 wherein nucleic acid encoding the IPNV coding regions is isolated using forward and reverse primer pairs specific for VP3 or VP2, wherein the primer pairs are selected from:
CTA ACA ACG GAA TTC ATG GAC AAA GTC VP2 forward primer (SEQ ID. NO. 1)
GAAGCTGCAGAGGACAAAGTCAAC VP2var forward (SEQ ID. NO. 2)
CGT TGC CGA TTG GCG GCC GCT GGT TGA TC VP2 reverse primer (SEQ ID. NO. 3)
ACCACTGCAGTCACAGTCCTGAATC VP2 forward (SEQ ID. NO. 4)
GAGCGCGGCCGCCGCAATTCCGTTCCCTG VP2 reverse (SEQ ID. NO. 5)
CCT GGG ACT GCA GAT GGC ATC AAA TG VP3 forward primer (SEQ ID. NO. 6)
GTT ACA CCG CGG CCG CGT CTC CGC TGG G VP3 reverse primer (SEQ ID. NO. 7)
GACGCTGCAGTGCAACGCCTCCTG VP3 forward (SEQ ID. NO. 8)
GTGCAGCGGCCGCCGGGGGTCGTCGTTTCATC VP3reverse (SEQ ID. NO. 9)
GACGCTGCAGTGCAACGCCTCCTG VP3 forward (SEQ ID. NO. 10)
CTCTCTAGAGTCTCCGCTGGG VP3 reverse (SEQ ID. NO. 11)
CCCTCAGAGTCACAGTCCTG VP2 forward (SEQ ID. NO. 12)
GAGCGCGGCCGCCGCAATTCCGTTCCCTG VP2 reverse (SEQ ID. NO. 13)

12. A process as claimed in any one of claims 1 to 11 wherein the or each IPNV polypeptide is attached to a linker polypeptide adapted to link the polypeptide to a microparticle.

13. A process as claimed in claim 12 further comprising the step of formulating the or each IPNV polypeptide to render it suitable for administration by immersion or orally via incorporation into fish food by packaging it within a micro-particulate delivery system selected from: latex bead; poly(lactide-co-glycolide) microspheres; atelocollagen minipellets; bentonite; or porous apatite ceramics.

14. A process as claimed in any one of the preceding claims which further comprises the step of combining the vaccine with other bacterial antigens to control other diseases.

15. A yeast expression vector, **characterised in that** the vector encodes two different IPNV polypeptides which are:
(a) an IPNV VP3 polypeptide or a fragment thereof comprising a sequence having at least 90% sequence identity with the sequence of Annex B or Annex D
(b) an IPNV VP2 polypeptide or a fragment thereof comprising a sequence having at least 90% sequence identity with the sequence of Annex A or Annex C,
in each case fused to a secretion signal sequence,
and wherein in each case the polypeptide or fragment is capable of stimulating the production of antibodies which bind IPNV.

16. A yeast host cell containing or transformed with the vector of claim 15.

17. A bivalent vaccine for use against IPNV in fish, which vaccine comprises supernatant from a yeast host cell comprising two different IPNV polypeptides
which are respectively:
(a) an IPNV VP3 polypeptide or a fragment thereof comprising a sequence having at least 90% sequence identity with the sequence of Annex B or Annex D
(b) an IPNV VP2 polypeptide or a fragment thereof comprising a sequence having at least 90% sequence identity with the sequence of Annex A or Annex C,
wherein in each case the polypeptide or fragment is capable of stimulating the production of antibodies which bind IPNV.

18. A vaccine as claimed in claim 17, **characterised in that** it consists essentially of the IPNV polypeptides VP3 having the sequence of Annex B or Annex D and VP2 having the sequence of Annex A or Annex C.

19. A vaccine as claimed in claim 17, **characterised in that** it consists essentially of the IPNV polypeptides VP3 having the sequence of Annex B or Annex D and VP2 having the sequence of Annex A or Annex C, in each case fused to a yeast secretion signal sequence.

20. A vaccine as claimed in claim 18 or claim 19 wherein the VP3 and VP2 sequences are shown in Annex B and Annex A.

21. A vaccine as claimed in claim 18 or claim 19 wherein the VP3 and VP2 sequences are shown in Annex D and Annex C.

22. A vaccine as claimed in claim 18 wherein the VP3 and VP2 sequences are shown in Annex E.

23. A vaccine as claimed in any one of claims 17 to 22 comprising a pharmacologically acceptable diluent or adjuvant or combination of these.

24. A vaccine as claimed in claim 23 which is selected from: an aqueous suspension for use as an immersion vaccine; an oral vaccine comprising fish oil and a lecithin carrier.

25. A vaccine composition comprising a vaccine as claimed in any one of claims 18 to 24 and other bacterial antigens used to control other diseases.

26. A vaccine composition as claimed in claim 25 wherein the antigens to other fish diseases are derived from *Aeromonas salmonicida* and\or are one or more *Vibrio* antigens derived from any of *V.anguillarum, V. salmonicida* and *V. viscosus.*

27. A vaccine or vaccine composition as claimed in any one of claims 18 to 26 for use in a method of therapeutic treatment, or prophylaxis, of IPNV in a fish, which method of therapeutic treatment or prophylaxis of IPNV comprises administering a dose of the IPNV vaccine to a fish.

28. A vaccine as claimed in claim 27 wherein the treatment or prophylaxis is against a strain of the Sp serotype of IPNV.

## Patentansprüche

1. Verfahren zur Herstellung eines zweiwertigen Impfstoffs zur Verwendung gegen infektiöses Pankreasnekrosevirus (IPNV) bei Fischen, **dadurch gekennzeichnet, dass** es Folgendes umfasst:
(i) das Kultivieren von Hefewirtszellen, die zwei verschiedene IPNV-Polypeptide umfassen, wovon
(a) das eine ein IPNV-VP3-Polypeptid oder ein Fragment davon ist, das eine Sequenz mit zumindest 90 % Sequenzidentität mit der Sequenz von Anhang B oder Anhang D umfasst;
(b) das andere ein IPNV-VP2-Polypeptid oder ein Fragment davon ist, das eine Sequenz mit zumindest 90 % Sequenzidentität mit der Sequenz von Anhang A oder Anhang C umfasst;
worin in beiden Fällen das Polypeptid oder Fragment in der Lage ist, die Produktion von Antikörpern zu stimulieren, die IPNV binden,
und worin die exprimierten IPNV-Polypeptide aus den Wirtszellen in den Kulturüberstand sekretiert werden;
(ii) das Formulieren der IPNV-Polypeptide als Impfstoff.

2. Verfahren nach Anspruch 1, worin die Hefewirtszelle Pichia pastoris ist.

3. Verfahren nach Anspruch 1, worin Wirtszellen aus dem Überstand herausgetrennt werden und der Überstand, der sekretierte IPNV-Polypeptide enthält, als der Impfstoff formuliert wird.

4. Verfahren nach Anspruch 1 oder Anspruch 3, worin die IPNV-Polypeptide mit einer Signalsequenz sekretiert werden, bei der es sich um die α-Hefepaarungsfaktorsignalsequenz handelt.

5. Verfahren nach einem der vorangegangenen Ansprüche, worin der Impfstoff durch Verdünnung mit PBS oder Zusatz von Adjuvans oder einer Kombination aus diesen formuliert wird.

6. Verfahren nach einem der Ansprüche 1 bis 5, das die folgenden Schritte umfasst:
(i) das Isolieren einer IPNV-Nucleinsäure-Kodierregion, die für Folgendes kodiert:
(a) ein IPNV-VP3-Polypeptid oder ein Fragment davon, das eine Sequenz mit zumindest 90 % Sequenzidentität mit der Sequenz von Anhang B oder Anhang D umfasst;
(b) ein IPNV-VP2-Polypeptid oder ein Fragment davon, das eine Sequenz mit zumindest 90 % Sequenzidentität mit der Sequenz von Anhang A oder Anhang C umfasst;
worin in beiden Fällen das Polypeptid oder Fragment in der Lage ist, die Produktion von Antikörpern zu stimulieren, die IPNV binden,
(ii) das Herstellen eines rekombinanten DNA-Plasmids, das die IPNV-Kodierregion enthält,
(iii) das Herstellen von Hefezelllinien, die das IPNV-Polypeptid exprimieren,
(iv) das Screenen auf Expression des IPNV-Polypeptids in den Zelllinien.

7. Verfahren nach einem der Ansprüche 1 bis 6, worin für die IPNV-Polypeptide mehrere Kopien der Kodierregion von IPNV-Genen kodieren, die zusammenligiert sind und so ein einziges offenes Leseraster mit einem einzigen Initiations- und Terminationscodon bilden, um ein mehrwertiges IPNV-Antigen hervorzubringen.

8. Verfahren nach einem der Ansprüche 1 bis 6, worin die IPNV-Polypeptide in verschiedenen Hefewirtszellen exprimiert werden.

9. Verfahren nach einem der Ansprüche 1 bis 8, worin jedes IPNV-Polypeptid als zwei oder mehrere Kopien von Antigen, die in der für die Expression als ein einziges Polypeptid richtigen Ausrichtung zusammenfusioniert sind, vorliegt, um so multimere IPNV-Antigene hervorzubringen.

10. Verfahren nach einem der Ansprüche 1 bis 6, worin die IPNV-Polypeptide VP3 mit der Sequenz von Anhang B oder Anhang D und VP2 mit der Sequenz von Anhang A oder Anhang C sind.

11. Verfahren nach Anspruch 10, worin Nucleinsäure, die für die IPNV-Kodierregionen kodiert, unter Verwendung von Vorwärts- und Rückwärtsprimerpaaren, die für VP3 oder VP2 spezifisch sind, isoliert wird, worin die Primerpaare aus den folgenden ausgewählt sind:
CTA ACA ACG GAA TTC ATG GAC AAA GTC: VP2-Vorwärtsprimer (Seq.-ID Nr. 1)
GAAGCTGCAGAGGACAAAGTCAAC: VP2var-Vorwärts (Seq.-ID Nr. 2)
CGT TGC CGA TTG GCG GCC GCT GGT TGA TC: VP2-Rückwärtsprimer (Seq.-ID Nr. 3)
ACCACTGCAGTCACAGTCCTGAATC: VP2-Vorwärts (Seq.-ID Nr. 4)
GAGCGCGGCCGCCGCAATTCCGTTCCCTG: VP2-Rückwärts (Seq.-ID Nr. 5)
CCT GGG ACT GCA GAT GGC ATC AAA TG: VP3-Vorwärtsprimer (Seq.-ID Nr. 6)
GTT ACA CCG CGG CCG CGT CTC CGC TGG G: VP3-Rückwärtsprimer (Seq.-ID Nr. 7)
GACGCTGCAGTGCAACGCCTCCTG: VP3-Vorwärts (Seq.-ID Nr. 8)
GTGCAGCGGCCGCCGGGGGTCGTCGTTTCATC: VP3-Rückwärts (Seq.-ID Nr. 9)
GACGCTGCAGTGCAACGCCTCCTG: VP3-Vorwärts (Seq.-ID Nr. 10)
CTCTCTAGAGTCTCCGCTGGG: VP3-Rückwärts (Seq.-ID Nr. 11)
CCCTCAGAGTCACAGTCCTG: VP2-Vorwärts (Seq.-ID Nr. 12)
GAGCGCGGCCGCCGCAATTCCGTTCCCTG: VP2-Rückwärts (Seq.-ID Nr. 13)

12. Verfahren nach einem der Ansprüche 1 bis 11, worin das oder jedes IPNV-Polypeptid an ein Linkerpolypeptid gebunden ist, das so angepasst ist, dass es das Polypeptid mit einem Mikropartikel verbindet.

13. Verfahren nach Anspruch 12, weiters umfassend den Schritt des Formulierens des oder jedes IPNV-Polypeptids in einer Weise, dass es sich zur Verabreichung durch Immersion oder zur oralen Verabreichung eignet, durch Einarbeitung in das Fischfutter, indem es in ein aus Latexkügelchen, Poly(lactidcoglykolid)-Mikrokugeln, Atelocollagenminipellets, Bentonit und poröser Apatitkeramik ausgewähltes mikropartikelförmiges Zufuhrsystem verpackt wird.

14. Verfahren nach einem der vorangegangenen Ansprüche, weiters umfassend den Schritt des Kombinierens des Impfstoffs mit weiteren bakteriellen Antigenen, um weitere Krankheiten zu bekämpfen.

15. Hefeexpressionsvektor, **dadurch gekennzeichnet, dass** der Vektor für zwei verschiedene IPNV-Polypeptide kodiert, wovon:
(a) das eine ein IPNV-VP3-Polypeptid oder ein Fragment davon ist, das eine Sequenz mit zumindest 90 % Sequenzidentität mit der Sequenz von Anhang B oder Anhang D umfasst;
(b) das andere ein IPNV-VP2-Polypeptid oder ein Fragment davon ist, das eine Sequenz mit zumindest 90 % Sequenzidentität mit der Sequenz von Anhang A oder Anhang C umfasst;
und die beide an eine Sekretionssignalsequenz fusioniert sind,
und worin in beiden Fällen das Polypeptid oder Fragment in der Lage ist, die Produktion von Antikörpern zu stimulieren, die IPNV binden.

16. Hefewirtszelle, die den Vektor nach Anspruch 15 enthält oder damit transformiert ist.

17. Zweiwertiger Impfstoff zur Verwendung gegen IPNV bei Fischen, wobei der Impfstoff Überstand aus einer Hefewirtszelle umfasst, die zwei verschiedene IPNV-Polypeptide umfasst, wovon:
(a) das eine ein IPNV-VP3-Polypeptid oder ein Fragment davon ist, das eine Sequenz mit zumindest 90 % Sequenzidentität mit der Sequenz von Anhang B oder Anhang D umfasst;
(b) das andere ein IPNV-VP2-Polypeptid oder ein Fragment davon ist, das eine Sequenz mit zumindest 90 % Sequenzidentität mit der Sequenz von Anhang A oder Anhang C umfasst;
worin in beiden Fällen das Polypeptid oder Fragment in der Lage ist, die Produktion von Antikörpern zu stimulieren, die IPNV binden.

18. Impfstoff nach Anspruch 17, **dadurch gekennzeichnet, dass** er im Wesentlichen aus den IPNV-Polypeptiden VP3 mit der Sequenz von Anhang B oder Anhang D und VP2 mit der Sequenz von Anhang A oder Anhang C besteht.

19. Impfstoff nach Anspruch 17, **dadurch gekennzeichnet, dass** er im Wesentlichen aus den IPNV-Polypeptiden VP3 mit der Sequenz von Anhang B oder Anhang D und VP2 mit der Sequenz von Anhang A oder Anhang C besteht, die beide an eine Hefesekretionssignalsequenz fusioniert sind.

20. Impfstoff nach Anspruch 18 oder Anspruch 19, worin die Sequenzen von VP3 bzw. VP2 in Anhang B bzw. Anhang A dargestellt sind.

21. Impfstoff nach Anspruch 18 oder Anspruch 19, worin die Sequenzen von VP3 bzw. VP2 in Anhang D bzw. Anhang C dargestellt sind.

22. Impfstoff nach Anspruch 18, worin die Sequenzen von VP3 und VP2 in Anhang E dargestellt sind.

23. Impfstoff nach einem der Ansprüche 17 bis 22, umfassend ein pharmakologisch annehmbares Verdünnungsmittel oder Adjuvans oder eine Kombination aus diesen.

24. Impfstoff nach Anspruch 23, der aus einer wässrigen Suspension zur Verwendung als Immersionsimpfstoff; einem Fischöl und Lecithinträger umfassenden oralen Impfstoff ausgewählt ist.

25. Impfstoffzusammensetzung, umfassend einen Impfstoff nach einem der Ansprüche 18 bis 24 und weitere zur Bekämpfung weiterer Krankheiten verwendete bakterielle Antigene.

26. Impfstoffzusammensetzung nach Anspruch 25, worin die Antigene gegen andere Fischkrankheiten von Aeromonas salmonicide stammen und/oder ein oder mehrere Vibrio-Antigene sind, die von einem beliebigen von V. anguillarum, V. salmonicida und V. viscosus stammen.

27. Impfstoff oder Impfstoffzusammensetzung nach einem der Ansprüche 18 bis 26 zur Verwendung in einem Verfahren zur therapeutischen Behandlung oder Prophylaxe von IPNV bei Fischen, wobei das Verfahren zur therapeutischen Behandlung oder Prophylaxe von IPNV das Verabreichen einer Dosis des IPNV-Impfstoffs an einen Fisch umfasst.

28. Impfstoff nach Anspruch 27, worin die Behandlung oder Prophylaxe gegen einen Stamm des Sp-Serotyps von IPNV gerichtet ist.

## Revendications

1. Procédé pour produire un vaccin bivalent pour utilisation contre le virus de nécrose pancréatique infectieuse (IPNV) dans des poissons, **caractérisé en ce qu'**il comprend:
(i) cultiver des cellules hôtes de levure qui expriment deux polypeptides IPNV différents qui sont respectivement:
(a) un polypeptide IPNV VP3 ou un fragment de celui-ci comprenant une séquence ayant au moins 90% d'identité de séquence avec la séquence de l'Annexe B ou de l'Annexe D,
(b) un polypeptide IPNV VP2 ou un fragment de celui-ci comprenant une séquence ayant au moins 90% d'identité de séquence avec la séquence de l'Annexe A ou de l'Annexe C, dans lequel dans chaque cas, le polypeptide ou fragment est apte à stimuler la production d'anticorps qui lient IPNV,
et où les polypeptides IPNV exprimés sont sécrétés des cellules hôtes dans le surnageant de culture, (ii) formuler les polypeptides IPNV comme un vaccin.

2. Procédé selon la revendication 1, dans lequel la cellule hôte de levure est *Pichia pastoris.*

3. Procédé selon la revendication 1, dans lequel les cellules hôtes sont séparées du surnageant, et le surnageant contenant des polypeptides IPNV sécrétés est formulé comme le vaccin.

4. Procédé selon la revendication 1 ou la revendication 3, dans lequel les polypeptides IPNV sont sécrétés avec une séquence signal qui est la séquence signal du facteur mat α de la levure.

5. Procédé selon l'une quelconque des revendications précédentes, dans lequel le vaccin est formulé par dilution avec PBS ou addition de l'adjuvant ou une combinaison de celles-ci.

6. Procédé selon l'une quelconque des revendications 1 à 5, qui comprend les étapes de:
(i) isoler une région de codage IPNV d'acide nucléique codant pour:
(a) un polypeptide IPNV VP3 ou un fragment de celui-ci comprenant une séquence ayant au moins 90% d'identité de séquence avec la séquence de l'Annexe B ou de l'Annexe D, ou
(b) un polypeptide IPNV VP2 ou un fragment de celui-ci comprenant une séquence ayant au moins 90% d'identité de séquence avec la séquence de l'Annexe A ou de l'Annexe C,
où dans chaque cas le polypeptide ou fragment est apte à stimuler la production d'anticorps qui lient IPNV, (ii) préparer un plasmide d'ADN recombinant contenant la région de codage IPNV,
(iii) préparer des lignées cellulaires de levure exprimant le polypeptide IPNV,
(iv) cribler l'expression du polypeptide IPNV dans les lignées cellulaires.

7. Procédé selon l'une quelconque des revendications 1 à 6, dans lequel les polypeptides IPNV sont codés par des copies multiples de la région de codage de gènes IPNV réunis par ligature pour former un cadre de lecture ouvert unique avec un seul codon d'initiation et de terminaison pour produire un antigène IPNV multivalent.

8. Procédé selon l'une quelconque des revendications 1 à 6, dans lequel les polypeptides IPNV sont exprimés dans des cellules hôtes de levure différentes.

9. Procédé selon l'une quelconque des revendications 1 à 8, dans lequel chaque polypeptide IPNV est présent sous forme de deux ou plusieurs copies d'antigène fusionnées ensemble dans l'orientation correcte pour l'expression comme un seul polypeptide de manière à produire des antigènes IPNV multimères.

10. Procédé selon l'une quelconque des revendications 1 à 6, dans lequel les polypeptides IPNV sont VP3 ayant la séquence de l'Annexe B ou de l'Annexe D, et VP2 ayant la séquence de l'Annexe A ou de l'Annexe C.

11. Procédé selon la revendication 10, dans lequel l'acide nucléique codant pour les régions de codage IPNV est isolé en utilisant des paires d'amorces avant et arrière spécifiques pour VP3 ou VP2, où les paires d'amorces sont sélectionnées parmi:
CTA ACA ACG GAA TTC ATG GAC AAA GTC VP2 amorce avant (SEQ ID. NO. 1)
GAAGCTGCAGAGGACAAAGTCAAC VP2var avant (SEQ ID. NO. 2)
CGT TGC CGA TTG GCG GCC GCT GGT TGA TC VP2 amoroe arrière (SEQ ID. NO. 3)
ACCACTGCAGTCACAGTCCTGAATC VP2 avant (SEQ ID. NO. 4)
GAGCGCGGCCGCCGCAATTCCGTTCCCTG VP2 arrière (SEQ ID. NO. 5)
CCT GGG ACT GCA GAT GGC ATC AAA TG VP3 amorce avant (SEQ ID. NO. 6)
GTT ACA CCG CGG CCG CGT CTC CGC TGG G VP3 amorce arrière (SEQ ID. NO. 7)
GACGCTGCAGTGCAACGCCTCCTG VP3 avant (SEQ ID. NO. 8)
GTGCAGCGGCCGCCGGGGGTCGTCGTTTCATC VP3 arrière (SEQ ID. NO. 9)
GACGCTGCAGTGCAACGCCTCCTG VP3 avant (SEQ ID. NO. 10)
CTCTCTAGAGTCTCCGCTGGG VP3 arrière (SEQ ID. ID. 11)
CCCTCAGAGTCACAGTCCTG VP2 avant (SEQ ID. NO. 12)
GAGCGCGGCCGCCGCAATTCCGTTCCCTG VP2 arrière (SEQ ID. NO. 13)

12. Procédé selon l'une quelconque des revendications 1 à 11, dans lequel le ou chaque polypeptide IPNV est attaché à un polypeptide lieur apte à lier le polypeptide à une microparticule.

13. Procédé selon la revendication 12, comprenant en outre l'étape consistant à formuler le ou chaque polypeptide IPNV pour qu'il convienne pour l'administration par immersion ou oralement par incorporation dans des aliments pour poissons en l'emballant dans un système de délivrance de micro-particules sélectionné parmi: perles de latex; microsphères poly(lactide-co-glycolide); mini-pastilles d'atélocollagène; bentonite; ou céramique d'apatite poreuse.

14. Procédé selon l'une quelconque des revendications précédentes, qui comprend en outre l'étape consistant à combiner le vaccin avec d'autres antigènes bactériens pour contrôler d'autres maladies.

15. Vecteur d'expression de levure, **caractérisé en ce que** le vecteur code deux polypeptides IPNV différents qui sont:
(a) un polypeptide IPNV VP3 ou un fragment de celui-ci comprenant une séquence ayant au moins 90% d'identité de séquence avec la séquence de l'Annexe B ou de l'Annexe D
(b) un polypeptide IPNV VP2 ou un fragment de celui-ci comprenant une séquence ayant au moins 90% d'identité de séquence avec la séquence de l'Annexe A ou de l'Annexe C, dans chaque cas fusionné à une séquence signal de sécrétion,
et où dans chaque cas, le polypeptide ou fragment est apte à stimuler la production d'anticorps qui lient IPNV.

16. Cellule hôte de levure contenant ou transformée avec le vecteur de la revendication 15.

17. Vaccin bivalent pour utilisation contre IPNV chez les poissons, ledit vaccin comprend le surnageant d'une cellule hôte de levure comprenant deux polypeptides IPNV différents, qui sont respectivement:
(a) un polypeptide IPNV VP3 ou un fragment de celui-ci comprenant une séquence ayant au moins 90% d'identité de séquence avec la séquence de l'Annexe B ou de l'Annexe D
(b) un polypeptide IPNV VP2 ou un fragment de celui-ci comprenant une séquence ayant au moins 90% d'identité de séquence avec la séquence de l'Annexe A ou de l'Annexe C, où dans chaque cas, le polypeptide ou fragment est apte à stimuler la production d'anticorps qui lient IPNV.

18. Vaccin selon la revendication 17, **caractérisé en ce qu'**il consiste essentiellement en polypeptides IPNV VP3 ayant la séquence de l'Annexe B ou de l'Annexe D et de VP2 ayant la séquence de l'Annexe A ou de l'Annexe C.

19. Vaccin selon la revendication 17, **caractérisé en ce qu'**il consiste essentiellement en polypeptides IPNV VP3 ayant la séquence de l'Annexe B ou de l'Annexe D et de VP2 ayant la séquence de l'Annexe A ou de l'Annexe C, dans chaque cas fusionnés à une séquence signal de sécrétion de levure.

20. Vaccin selon la revendication 18 ou la revendication 19, dans lequel les séquences VP3 et VP2 sont représentées dans l'Annexe B et dans l'Annexe A.

21. Vaccin selon la revendication 18 ou la revendication 19, dans lequel les séquences VP3 et VP2 sont représentées dans l'Annexe D et dans l'Annexe C.

22. Vaccin selon la revendication 18, dans lequel les séquences VP3 et VP2 sont représentées dans l'Annexe E.

23. Vaccin selon l'une quelconque des revendications 17 à 22, comprenant un diluant ou adjuvant pharmacologiquement acceptable ou une combinaison de ceux-ci.

24. Vaccin selon la revendication 23, qui est sélectionné parmi: une suspension aqueuse pour utilisation comme vaccin d'immersion; un vaccin oral comprenant de l'huile de poisson et un support de lécithine.

25. Composition de vaccin comprenant un vaccin tel que revendiqué dans l'une quelconque des revendications 18 à 24 et d'autres antigènes bactériens utilisés pour contrôler d'autres maladies.

26. Composition de vaccin selon la revendication 25, dans laquelle les antigènes à d'autres maladies des poissons sont dérivés de *Aeromonas salmonicida* et/ou sont un ou plusieurs Vibrio antigènes dérivés d'un quelconque de *V. anguillarum, V. salmonicida* et *V. viscosus.*

27. Vaccin ou composition de vaccin selon l'une quelconque des revendications 18 à 26 pour utilisation dans une méthode de traitement thérapeutique, ou prophylaxie, de IPNV dans un poisson, ladite méthode de traitement ou prophylaxie thérapeutique de IPNV comprend l'administration d'une dose du vaccin IPNV à un poisson.

28. Vaccin selon la revendication 27, dans lequel le traitement ou la prophylaxie est contre une souche du sérotype Sp de IPNV.
